# EUROPÄISCHE PATENTSCHRIFT

(11) **EP 3 057 556 B1**
(45) Veröffentlichungstag und Bekanntmachung des Hinweises auf die Patenterteilung: **28.03.2018**
(21) Anmeldenummer: 14796721.0
(22) Anmeldetag: 17.10.2014
(51) Int. Cl.: A61G 13/10, A61G 13/12

(54) **BEFESTIGUNGSEINHEIT ZUR BEFESTIGUNG EINER VORRICHTUNG ZUM LAGERN EINES ZU RÖNTGENDEN PATIENTEN AN EINEM OPERATIONSTISCH**
FASTENING UNIT FOR FASTENING A DEVICE FOR SUPPORTING A PATIENT TO BE X-RAYED TO AN OPERATING TABLE
UNITÉ DE FIXATION PERMETTANT DE FIXER UN DISPOSITIF DESTINÉ AU POSITIONNEMENT D'UN PATIENT À RADIOGRAPHIER SUR UNE TABLE D'OPÉRATION

(30) Priorität: 18.10.2013 DE 102013111522
(43) Veröffentlichungstag der Anmeldung: 24.08.2016
(73) Patentinhaber: Maquet GmbH, 76437 Rastatt (DE)
(72) Erfinder: HAFNER, Dieter, 77654 Offenburg (DE); HUND, Siegfried, 77704 Oberkirch (DE); WYSLUCHA, Ulrich, 76356 Weingarten (DE)
(74) Vertreter: Zacco GmbH
(86) Internationale Anmeldenummer: PCT/EP2014/072322
(87) Internationale Veröffentlichungsnummer: WO 2015/055817

(56) Entgegenhaltungen:
- EP-A1- 2 325 502
- EP-A2- 1 785 123
- WO-A1-2009/029524
- WO-A1-2013/069952

## Beschreibung

Die Erfindung betrifft eine operationstischseitige Befestigungseinheit zur Befestigung einer Vorrichtung zum Lagern eines zu röntgenden Patienten an einem Operationstisch, die einen Grundkörper umfasst, der eine Aufnahme zur Aufnahme eines Koppelelements einer an der Vorrichtung befestigbaren vorrichtungsseitigen Befestigungseinheit hat. An dem Grundkörper ist relativ zu diesem bewegbar ein Riegel befestigt, wobei der Riegel in einer verriegelten Position ein Entfernen des Koppelelements aus der Aufnahme verhindert und in einer entriegelten Position ein Entfernen des Koppelelements aus der Aufnahme erlaubt. Ferner betrifft die Erfindung eine vorrichtungsseitige Befestigungseinheit zur Befestigung einer Vorrichtung zum Lagern eines zu röntgenden Patienten an einem Operationstisch, die ein Zwischenstück umfasst, das ein Koppelelement zum Einführen in eine Aufnahme der operationstischseitigen Befestigungseinheit hat. Darüber hinaus betrifft die Erfindung eine Anordnung, die einen Operationstischtisch, eine Vorrichtung zur Lagern eines zu röntgenden Patienten sowie operationstischseitige und vorrichtungsseitige Befestigungseinheiten zur Befestigung der Vorrichtung an dem Operationstisch aufweist.

Bei verschiedenen Operationen, insbesondere bei Operationen an der Wirbelsäule, ist es notwendig, dass der Patient während der Operation geröntgt werden kann. Hierzu werden häufig C-förmige Röntgenapparate verwendet, wobei in der Öffnung des "C" der Patient gelagert ist. Insbesondere werden auch um bis zu 270° verschwenkbare C-Bögen zur Aufnahme von 3D-Bildern verwendet. Die Verwendung von klassischen Operationstischen zur Lagerung des Patienten ist nicht oder nur eingeschränkt möglich, da ein Röntgen nur in sehr beschränktem Umfang möglich ist. Zur Aufnahme von 3D-Aufnahmen muss der C-Bogen möglichst nah am Patienten über einen möglichst großen Bereich bewegt werden können. Die standardmäßigen Patientenauflagen von Operationstischen sind hierfür zu breit ausgebildet. Zum anderen umfasst der Operationstisch häufig dicke, metallhaltige Konstruktionen, so dass ein Röntgen nur in unzureichender Qualität möglich ist. Erschwerend kommt hinzu, dass die Dicke und Konturen der Konstruktionen sehr unterschiedlich sind, was die Qualität des Röntgenbildes weiter negativ beeinflusst und die Röntgenaufnahme über den C-Bogen limitiert.

Daher werden Vorrichtungen zum Lagern des Patienten verwendet, die an den Operationstisch angebaut werden können. Der Patient liegt dann mit dem Oberkörper, an dem operiert wird und der geröntgt werden muss, auf der angebauten Vorrichtung und lediglich mit seinen Beinen auf dem eigentlichen Operationstisch.

Eine solche Vorrichtung zum Lagern eines Patienten während einer Operation ist beispielsweise aus dem Dokument US 7,600,281 B2 bekannt. Die dort beschriebene Vorrichtung umfasst zwei parallel zueinander verlaufende Holme, die an der einen Seite am Operationstisch und an der anderen Seite an einem Ständer befestigt sind. An den Holmen sind mehrere Auflageflächen vorgesehen, auf denen der Patient, insbesondere der Oberkörper und die Hüften gelagert werden können. Diese Auflageflächen ragen hierbei über den gesamten Bereich zwischen den beiden Holmen.

Die zuvor beschriebene Vorrichtung hat den Nachteil, dass durch die Auflageelemente die Qualität eines aufgenommenen Röntgenbildes negativ beeinflusst werden kann. Die Konturen der Auflageelemente finden sich im Röntgenbild wieder, was ggf. zu Fehlinterpretationen führen kann. Darüber hinaus ermöglichen die starren Auflageelemente nur eine unzureichende Anpassung an die individuellen Konturen des Patienten, so dass dieser ggf. nicht optimal für die Operation gelagert werden kann. Bei den bekannten Vorrichtungen werden die Schienen über die zwei Schnittstellen am Operationstisch befestigt, wobei zwischen den beiden Schnittstellen eine Querverbindung besteht, indem ein Bedienelement angeordnet ist, welches den Vorteil hat, dass beide Schnittstellen mit Hilfe nur eines Bedienelementes entriegelt werden können, so dass die Vorrichtung von dem Operationstisch gelöst werden kann. Zusätzlich kann eine weitere metallische Querverbindung als Rahmen vorgesehen sein. Dies hat zum einen den Nachteil, dass in diesem Bereich die Durchlässigkeit von Röntgenstrahlen nur begrenzt möglich ist, was zu qualitativ schlechten Röntgenbildern führen kann, und zum anderen, dass die Verstellmöglichkeiten des Operationstisches und der Patientenlagereinheiten zueinander nur sehr begrenzt sind. Insbesondere ist eine Höhenverstellung zwischen den Schienen und dem Operationstisch nicht möglich und ein Abwinkeln der Befestigungsanordnungen relativ zum Operationstisch auf ungefähr 50° begrenzt. Eine weitere Vorrichtung zum Lagern eines Patienten ist aus WO2013/069952 A1 bekannt. Eine weitere vorrichtungsseitige Befestigungseinheit zur Befestigung einer Vorrichtung zum Lagern eines zu röntgenden Patienten während einer Operation an einem Operationstisch ist aus EP 2 325 502 A1 bekannt. Es ist Aufgabe der Erfindung, einen operationstischseitige Befestigungseinheit zur Befestigung einer Schiene einer Vorrichtung zum Lagern eines zu röntgenden Patienten an einem Operationstisch, eine vorrichtungsseitige Befestigungseinheit zur Befestigung einer Vorrichtung zum Lagern eines zu röntgenden Patienten an einem Operationstisch und eine Anordnung aus einem Operationstisch, einer Vorrichtung zum Lagern eines zu röntgenden Patienten und Befestigungseinheiten anzugeben, die bei einer einfachen Bedienbarkeit eine sichere Befestigung, größtmögliche Verstellmöglichkeiten und die Aufnahme qualitativ hochwertiger Röntgenbilder in einem möglichst großen Bereich ermöglichen.

Diese Aufgabe wird durch einen operationstischseitige Befestigungseinheit nach dem Anspruch 1, sowie eine Anordnung nach dem weiteren unabhängigen Anspruch 11 bzw. 12 gelöst. Vorteilhafte Weiterbildungen der Erfindung sind in den abhängigen Ansprüchen angegeben.

Erfindungsgemäß ist ein elastisches Element vorgesehen, das den Riegel in der Verriegelungsposition vorspannt. Mit Hilfe eines Betätigungselementes zum manuellen Bewegen des Riegels kann diese entgegen der elastischen Kraft des Vorspannelementes von der verriegelten in die entriegelte Position bewegt werden. Ferner umfasst die operationstischseitige Befestigungseinheit einen Gesperremechanismus zum Halten des Riegels in der entriegelten Position, wobei dieser Gesperremechanismus den Riegel, wenn ein Koppelelement in der Aufnahme angeordnet ist und der Riegel mit Hilfe des Betätigungselementes in der entriegelten Position oder über diese hinaus bewegt wurde, zumindest solange in der entriegelten Position hält, bis das Koppelelement aus der Aufnahme entfernt worden ist oder das Betätigungselement erneut betätigt worden ist.

Hierdurch wird erreicht, dass sowohl zum Lösen der vorrichtungsseitigen Befestigungseinheit, als auch zur Entnahme des Koppelelements aus der Aufnahme, das Betätigungselement nur einmal betätigt werden muss und anschließend der Riegel in der entriegelten Stellung gehalten ist, so dass eine Bedienperson beide Hände frei hat, um die Vorrichtung auszuhängen. Insbesondere bei der Verwendung von zwei operationstischseitigen Befestigungseinheiten an zwei parallel zueinander verlaufenden Schienen oder anderen Ausführungsform mit zwei Schnittstellen zur Befestigung an dem Operationstisch können diese somit bequem nacheinander gelöst werden. Durch das automatische Lösen des Gesperremechanismus, d.h. dadurch, dass der Gesperremechanismus den Riegel nicht länger in der entriegelten Position hält, wenn das Koppelelement aus der Aufnahme entfernt worden ist, wird erreicht, dass der Riegel automatisch wieder in der Verriegelungsposition angeordnet wird und somit beim Zuführen eines neuen Koppelelements in die Aufnahme automatisch in der verriegelten Position angeordnet ist und das Koppelelement zuverlässig in dieser hält.

Alternativ kann der Gesperremechanismus auch über eine erneute Betätigung des Betätigungselementes gelöst werden, was den Vorteil hat, dass bei einer unbeabsichtigten Betätigung des Betätigungselementes die verriegelte Position wieder hergestellt werden kann, ohne dass hierfür das Koppelelement aus der Aufnahme entfernt werden muss. Hierdurch werden die Sicherheit und der Bedienkomfort weiter erhöht.

Unter der Betätigung des Betätigungselements wird im Rahmen dieser Anmeldung insbesondere verstanden, dass das Betätigungselement vollständig bis an einen Anschlag betätigt wird.

Der Gesperremechanismus ist vorzugsweise als ein sogenannter Flip-Flop-Mechanismus ausgebildet. Alternativ kann der Gesperremechanismus auch z.B. über eine Führungskurve realisiert sein.

Die Vorrichtung zum Lagern umfasst insbesondere zwei Schienen, die jeweils über eine operationstischseitige und eine vorrichtungsseitige Befestigungseinheit an dem Operationstisch befestigt werden können. Die vorrichtungsseitige Befestigungseinheit wird dabei auch als schienenseitige Befestigungseinheit bezeichnet.

Alternativ kann die Vorrichtung auch nur eine Schiene mit Auslegern oder mehr als zwei Schienen, z.B. drei oder vier Schienen, umfassen. Ferner kann alternativ eine einstückige Struktur, insbesondere aus kohlefaserverstärktem Kunststoff, verwendet werden.

Das Koppelelement ist insbesondere als Stange ausgebildet. Alternativ können auch eine Kugel, ein über einen Haken geführter Ring oder jede andere Form, die ein Kippen der Vorrichtung zum Lagern des Patienten relativ zum OP-Tisch ermöglicht, verwendet werden.

Bei dem elastischen Element handelt es sich insbesondere um eine Drehfeder, deren erstes Ende sich an dem Grundkörper und deren zweites Ende sich an dem Riegel abstützt.

Der Riegel ist insbesondere derart im Grundkörper geführt, dass er sich auf einer zumindest annähernd kreisförmigen Bahn bewegt, weswegen der Riegel auch als Rotationsriegel bezeichnet wird und entsprechend insbesondere bogenförmig ausgebildet und an seine Führungsbahn angepasst ist.

Die Aufnahme ist vorzugsweise V-förmig ausgebildet, wodurch ein sicheres Halten des Koppelelements der vorrichtungsseitigen Befestigungseinheit in der Aufnahme erreicht wird. Der Bodenbereich der Aufnahme, also derjenige Bereich, der der Öffnung des "Vs" abgewandt ist, ist hierbei insbesondere abgerundet, vorzugsweise komplementär zu dem Koppelelement ausgebildet. Somit wird das Spiel zwischen der Aufnahme und dem Koppelelement minimiert, so dass eine sichere Lagerung gewährleistet ist.

Das Betätigungselement umfasst vorzugsweise einen an dem Riegel befestigten Zughebel, der derart ausgebildet und gelagert ist, dass er sowohl zum Bewegen des Riegels von der verriegelten in die entriegelte Position als auch zum Lösen des Gesperremechanismus, so dass der Riegel trotz aufgenommenem Koppelelement durch elastische Elemente zurück in die verriegelte Position bewegt wird, von dem Grundkörper weg gezogen werden muss. Hierdurch wird erreicht, dass der Zughebel immer nur auf die gleiche Art betätigt werden muss, egal ob der Riegel von der verriegelten in die entriegelte oder von der entriegelten in die verriegelte Position bewegt werden soll. Insbesondere ist der Zughebel der bei bestimmungsgemäßer Ausrichtung der Befestigungseinheit an deren unteren Seite angeordnet, so dass er nicht so leicht unbeabsichtigt betätigt werden kann und nicht "im Weg herumhängt". Insbesondere ist der Zughebel drehbar an dem Riegel angeordnet, so dass er platzsparend weggeklappt werden kann.

Bei einer bevorzugten Ausführungsform ist an dem Riegel ein Schwerkraftpendel drehbar befestigt, das aufgrund seiner Schwerkraft unabhängig von der Ausrichtung der Befestigungseinheit absolut im Raum betrachtet seine Ausrichtung beibehält. Das Schwerkraftpendel ist derart ausgebildet, dass es eine Betätigung des Betätigungselementes unterbindet, wenn die Befestigungseinheit außerhalb eines vorbestimmten Ausrichtungsbereiches relativ zur Horizontalen angeordnet ist. Die operationstischseitige Befestigungseinheit wird insbesondere in Aufnahmen des Operationstisches aufgenommen, wobei diese derart ausgebildet sind, dass die operationstischseitige Befestigungseinheit relativ zur Horizontalen sowohl nach oben als auch nach unten gekippt werden kann. Hierdurch wird eine möglichst große Verstellbarkeit erreicht, indem hierüber der Winkel zwischen der Vorrichtung und dem Operationstisch eingestellt werden kann.

Durch das Vorsehen eines Schwerkraftpendels wird sichergestellt, dass ein Lösen der Verriegelung nur dann möglich ist, wenn die operationsseitige Befestigungseinheit in etwa horizontal angeordnet ist, so dass insbesondere ein Entspannen dann verhindert wird, wenn die Befestigungseinheit so weit geneigt ist, dass ohne den Riegel des Koppelelements aufgrund ihrer Schwerkraft automatisch aus der Aufnahme herausrutschen würde.

Ferner ist es vorteilhaft, wenn das in die Aufnahme ragende Ende des Riegels, der, sofern der Riegel in der verriegelten Position angeordnet ist, ein Entfernen des Koppelelements aus der der Aufnahme verhindert, derart abgeschrägt ist, dass der Riegel beim Einführen des Koppelelements in die Aufnahme durch den Kontakt zum Koppelelement automatisch aus der verriegelten Position herausbewegt wird, so dass das Koppelelement in die Aufnahme eingeführt werden kann. Hierbei wird der Riegel jedoch nicht so weit in Richtung der entriegelten Position bewegt, dass er vollständig in der entriegelten Position angeordnet ist, also nicht so weit, dass der Gesperremechanismus den Riegel je nach Stellung des Gesperremechanismus festhalten könnte. Auf diese Weise wird eine besonders einfache Handhabung erreicht, da das Koppelement einfach nur in die Aufnahme hineingedrückt werden muss, ohne dass der Riegel hierfür extra betätigt wird. Auf der anderen Seite wird sichergestellt, dass der Riegel sich wieder automatisch in die verriegelte Position bewegt, so dass dieses nicht vergessen werden kann und eine hohe Sicherheit gegeben ist. So wird insbesondere vermieden, dass der Gesperremechanismus den Riegel unbeabsichtigt in der entriegelten Position festhält.

Bei einer besonders bevorzugten Ausführungsform umfasst die Befestigungseinheit einen drehbar an dem Grundkörper befestigten Taster zur Detektion der Aufnahme eines Koppelelements in der Aufnahme, der mit Hilfe eines weiteren elastischen Elementes in einer ersten Position vorgespannt ist, in der er zumindest teilweise in die Aufnahme hineinragt. In einer zweiten Position ist der Taster relativ zur ersten Position entgegen der Rückstellkraft des weiteren elastischen Elementes aus der Aufnahme heraus in den Grundkörper hineinbewegt. Der Taster ist dabei derart ausgebildet, dass er beim Einführen des Koppelelements in die Aufnahme automatisch von der ersten in die zweite Position bewegt und durch das aufgenommene Koppelelement in dieser zweiten Position solange gehalten wird, solange das Koppelelement in der Aufnahme angeordnet ist. Somit kann durch den Taster auf einfache Weise jederzeit mechanisch festgestellt werden, ob das Koppelelement in der Aufnahme aufgenommen ist. Hierüber kann insbesondere, wie im Folgenden noch näher erläutert, das automatische Lösen des Gesperremechanismus bei der Entnahme des Koppelelements aus der Aufnahme realisiert werden.

Darüber hinaus wird durch das Bewegen des Tasters von der ersten in die zweite Position insbesondere ein weiteres elastisches Element aus dem Grundkörper in die Richtung, an der der Operationstisch angeordnet ist, bewegt, durch das das Spiel zwischen der Befestigungseinheit und dem Operationstisch minimiert, vorzugsweise beseitigt wird, so dass eine wackelfreie Befestigung an dem Operationstisch erfolgt. Bei diesem weiteren elastischen Element handelt es sich insbesondere um einen Silikonblock.

Es ist vorteilhaft, wenn der Gesperremechanismus ein erstes Klinkenrad, ein zweites Klinkenrad und eine Vierkantscheibe umfasst, die jeweils drehfest auf einer gemeinsamen Welle gelagert sind. Ferner hat der Gesperremechanismus ein Sperrelement, das in einer Sperrposition, insbesondere durch eine Feder, vorgespannt ist. In dieser Sperrposition ist das Sperrelement mit dem zweiten Klinkenrad in Eingriff und verhindert somit eine Drehung des zweiten Klinkenrades in eine erste Richtung und daher auch eine Drehung der Welle und der auf ihr befestigten Elemente in die erste Richtung. Ist das Sperrelement dagegen entgegen der Vorspannung in der gelösten Position angeordnet, so ist eine Drehung der Welle in die erste Richtung möglich.

Das erste und das zweite Klinkenrad sind insbesondere derart ausgebildet, dass sie unabhängig von der Position des Sperrelementes jeweils eine Drehung entgegen der ersten Richtung erlauben.

Es ist vorteilhaft, wenn der Taster, sofern dieser in der ersten Position angeordnet ist, das Sperrelement kontaktiert und dieses über diesen Kontakt entgegen seiner Vorspannung in der gelösten Position hält. Ist der Taster dagegen in der zweiten Position angeordnet, so hält er das Sperrelement nicht mehr in der gelösten Position, so dass das Sperrelement aufgrund seiner Vorspannung sich in die Sperrposition bewegt und ein Drehen der Welle in die erste Richtung unterbindet. Mit diesem Mechanismus wird erreicht, dass eine Drehung der Welle in die erste Richtung nur dann möglich ist, wenn der Taster in der ersten Position angeordnet ist, also wenn kein Koppelelement in der Aufnahme angeordnet ist. Ist dagegen ein Koppelelement in der Aufnahme angeordnet, ist eine Drehung der Welle in die erste Richtung nicht möglich.

Der Riegel weist insbesondere einen Vorsprung auf, wobei der Vorsprung mit der Vierkantscheibe in Eingriff ist, wenn die Vierkantscheibe in einer Sperrwinkelstellung ausgerichtet ist, das Sperrelement in der Sperrposition angeordnet ist und wenn der Riegel mit Hilfe des Betätigungselementes über die entriegelte Position hinaus bewegt worden ist, so dass die Vierkantscheibe den Riegel in der entriegelten Position hält.

Wie bereits zuvor beschrieben, ist das Sperrelement, wenn ein Koppelelement in die Aufnahme eingeführt ist, in seiner Sperrposition angeordnet und unterbindet somit ein Drehen der Welle in die erste Richtung. Nach dem Einführen des Koppelelements ordnet sich der Riegel automatisch in der verriegelten Position an. Wird nur das Betätigungselement zum Entriegeln betätigt, so wird bei der Betätigung der Riegel zunächst über die entriegelte Position, in der er über die Vierkantscheibe des Gesperremechanismus gehalten werden kann, hinausbewegt. Sofern die Vierkantscheibe dann in einer Sperrwinkelstellung angeordnet ist, kommt der Vorsprung mit der Vierkantscheibe beim Zurückbewegen des Riegels in Eingriff. Die Welle müsste entgegen der ersten Richtung gedreht werden, damit die Vierkantscheibe sich aus der Sperrwinkelstellung drehen kann und das elastische Element des Riegelelementes diesen von der entriegelten in die verriegelte Position bewegen kann. Diese Drehung in die erste Richtung wird jedoch durch das in der Sperrposition angeordnete Sperrelement unterbunden, so dass der Gesperremechanismus den Riegel in der entriegelten Position hält. Wird das Koppelelement aus der Aufnahme entfernt, so bewegt sich der Taster in die erste Position, wodurch er das Sperrelement in die gelöste Position bewegt, so dass nun die Welle in die erste Richtung gedreht werden kann, was durch die durch das elastische Element des Riegels bewirkte Kraft auch passiert. Somit wird der Gesperremechanismus beim Entnehmen des Koppelelements automatisch gelöst und der Riegel von der entriegelten in die verriegelte Position bewegt.

Ist die Vierkantscheibe dagegen in eine Entsperrwinkelstellung angeordnet, so erlaubt sie ein Bewegen des Riegels von der entriegelten in die verriegelte Position unabhängig davon, ob das Sperrelement eine Drehung der Welle in die erste Richtung unterbindet oder nicht. Die Form des ersten Klinkenrades und der Vierkantscheibe sind insbesondere derart aufeinander abgestimmt, dass bei einer Drehung des ersten Klinkenrades um eine Klinke entgegen der ersten Drehrichtung die Ausrichtung der Vierkantscheibe sich jeweils zwischen der Sperrwinkelstellung und der Entsperrwinkelstellung verändert. Hierbei wird die Welle, und somit auch die Vierkantscheibe, immer in die gleiche Richtung, nämlich entgegen der ersten Richtung, bewegt.

Das erste Klinkenrad hat insbesondere die doppelte Anzahl an Klinken wie die Vierkantscheibe Ecken, also vorzugsweise acht Klinken.

An dem Riegel ist insbesondere ein Kontaktelement zur Kontaktierung des ersten Klinkenrades vorgesehen, wobei das Kontaktelement bei einer Betätigung des Betätigungselementes jeweils das erste Klinkenrad kontaktiert und über diesen Kontakt um eine Klinke entgegen der ersten Richtung dreht. Somit wird bei jeder Betätigung die Vierkantscheibe zwischen der Sperrwinkelstellung und der Entsperrwinkelstellung verändert. Dies hat insbesondere den Vorteil, dass, wenn der Riegel durch eine erste Betätigung des Betätigungselementes in die entriegelte Position bewegt wurde, trotz aufgenommenem Koppelelement in der Aufnahme der Riegel wieder in die verriegelte Position bewegt werden kann, indem einfach das Betätigungselement erneut betätigt wird.

Das Kontaktelement ist insbesondere relativ zum Riegel federnd gelagert, wobei es insbesondere in einer Aussparung des Riegels angeordnet ist und soweit bewegbar ist, dass es zumindest teilweise, vorzugweise vollständig, in den Riegel hineinbewegt werden kann. Dies hat den Vorteil, dass es bei einem Bewegen des Riegels in Richtung der verriegelten Stellung sich auf den abgeschrägten Seiten der Sperrklinken abstützen kann und somit in den Riegel hineinbewegt wird, und somit eine Bewegung des Riegels in Richtung der entriegelten Position nicht behindert.

Bei einer bevorzugten Ausführungsform hat die Befestigungseinheit eine Verbindungseinheit zur Befestigung der Befestigungseinheit an eine Beinplattenschnittstelle eines Operationstisches, wobei diese Verbindungseinheit einen Rastmechanismus zum Befestigen an dem Operationstisch umfasst. Es ist insbesondere ein weiteres, vorzugsweise ebenfalls an der Unterseite der Befestigungseinheit angeordnetes, Betätigungselement, insbesondere ein Knopf, zum Lösen des Rastmechanismus vorgesehen. Die Verbindungseinheit kann somit auf einfache Weise in ohnehin schon am Operationstisch vorgesehenen Schnittstellen befestigt werden. Insbesondere erlauben diese Schnittstellen auch eine Veränderung der Winkelstellung der Vorrichtung zum Lagern des Patienten relativ zum Operationstisch. Durch Veränderung der Winkelstellung der Vorrichtung kann auch die Höhe der Aufnahme zur Aufnahme des Koppelelements relativ zu einem Mittelteil des Operationstisches verändert werden.

Der Rastmechanismus umfasst insbesondere eine Kugel, die in einer komplementären Aussparung an dem Operationstisch einrastet. Ferner umfasst die Verbindungseinheit zusätzlich insbesondere einen Stift, der in einer Aussparung des Operationstisches eingeführt wird und eine Verdrehung der Befestigungseinheit unterbinden soll.

Darüber hinaus kann die Befestigungseinheit einen RFID-Chip umfassen, mit deren Hilfe sie eindeutig identifiziert werden kann.

Die zuvor beschriebenen operationstischseitigen Befestigungseinheiten haben den Vorteil, dass sie auf einfache Weise von einer Person ohne Zeitdruck bedient werden können. Da zwei Schnittstellen getrennt voneinander betätigt werden müssen, ist ferner eine Sicherheit gegen ein unbeabsichtigtes Lösen erreicht. Die Anordnung des Betätigungselementes an der Unterseite der Befestigungeinheit hat die Vorteile, dass es nicht störend in das Arbeitsfeld hineinragt, nicht anfällig für unbeabsichtigtes Auslösen ist und die gleiche Befestigungseinheit bei gleicher Bedienbarkeit sowohl für eine linke als auch eine rechte Schiene der Vorrichtung zur Lage des Patienten verwendet werden kann. Somit besteht keine Verwechselungsgefahr und es ist eine kostengünstige Fertigung gegeben.

Der beschrieben Gesperremechanismus ermöglicht ein Verriegeln und Entriegeln durch die gleiche Bedienung, d.h. durch ein Betätigen des Betätigungselementes in die gleiche Richtung. Die Betätigung als ein Ziehen nach unten ist ergonomisch besonders günstig.

Die automatische Verriegelung sowohl beim Ankoppeln als auch Entkoppeln, also sowohl beim Zuführen als auch Entnehmen des Koppelelements führt dazu, dass Fehlbedienungen durch den Anwender und ein zusätzlicher Arbeitsschritt vermieden werden.

Ein weiterer Aspekt der Erfindung betrifft eine vorrichtungsseitige Befestigungseinheit zur Befestigung einer Vorrichtung zum Lagern eines zu röntgenden Patienten an einem Operationstisch, die eine an der Vorrichtung befestigbare Montageeinheit und eine relativ zur Montageeinheit um eine Drehachse drehbar an dieser befestigtes Zwischenstück umfasst. An dem Zwischenstück ist ein Koppelelement zum Einführen in eine Aufnahme einer operationstischseitigen Befestigungseinheit vorgesehen. Ferner hat das Zwischenstück eine Platte, die insbesondere an einer Platte der Montageeinheit anliegt, wobei die beiden Platten sich aufeinander abstützen und ein Verdrehen relativ zueinander ermöglichen. Die Platte des Zwischenstückes weist zwei Aussparungen auf, die im gleichen Abstand zur Drehachse, jedoch an gegenüberliegenden Seiten der Drehachse, also insbesondere punktsymmetrisch an der Drehachse angeordnet sind. Auch die Platte der Montageeinheit weist eine Aussparung auf, die den gleichen Abstand zur Drehachse wie die beiden Aussparungen des Zwischenstücks hat. Das Koppelelement des Zwischenstücks ist versetzt zur Drehachse angeordnet, d.h. dass die Drehachse und die Längsachse des Koppelelements sich nicht schneiden, so dass, je nachdem, wie das Zwischenstück relativ zur Montageeinheit gedreht ist, das Koppelelement relativ zur Montageeinheit in einer unterschiedlichen Position, insbesondere in einer unterschiedlichen Höhe angeordnet ist, so dass dadurch eine einfache Höhenverstellung möglich ist.

Das Koppelelement ist insbesondere derart ausgebildet, dass sie komplementär zu der Aufnahme der zuvor beschriebenen operationstischseitigen Befestigungseinheit ausgebildet ist und in dieser aufnehmbar ist.

Das Koppelelement ist insbesondere als Stange, Kugel oder Ring ausgebildet.

Die Montageeinheit umfasst vorzugsweise eine Bolzenaufnahme, wobei das Zwischenstück einen Bolzen hat, der in dieser Aufnahme drehbar gelagert ist, wobei die Längsachse des Bolzens die Drehachse bildet. Auf der dem Zwischenstück abgewandten Ende des Bolzens, das aus der Bolzenaufnahme hinausragt, ist ein Element angeordnet, was ein Herausbewegen des Bolzens auf der Bolzenausnahme verhindert. Somit ist auf einfache Weise die Drehbarkeit zwischen der Montageeinheit und dem Zwischenstück gegeben und trotzdem das Zwischenstück unverlierbar an der Montageeinheit befestigt.

Bei einer besonders bevorzugten Ausführungsform ist in einer ersten Montageposition das Zwischenstück derart relativ zur Montageeinheit gedreht, dass sich die Aussparung der Platte der Montageeinheit mit einer der Aussparungen des Zwischenstückes überdeckt, d.h., dass diese koaxial zueinander angeordnet sind.

In einer zweiten Montageposition dagegen überdeckt sich die Aussparung der Platte der Montageeinheit mit der anderen Aussparung der Platte der Zwischeneinheit. Das Zwischenstück ist zwischen diesen beiden Montagepositionen insbesondere relativ zur Montageeinheit um 180° gedreht. Durch diese Anordnung des Koppelelements relativ zu der Drehachse wird somit erreicht, dass das Koppelelement in einer der beiden Montagepositionen bei unveränderter Ausrichtung der Montageeinheit überhalb der Drehachse und einmal unterhalb der Drehachse angeordnet ist, so dass hierdurch die relative Höhe zwischen der Vorrichtung und dem Operationstisch verändert werden kann. Insbesondere kann somit eine Höhenveränderung zwischen 5 und 9 cm, vorzugsweise um etwa 7 cm, erfolgen.

Die Montageeinheit umfasst insbesondere einen Federbolzen zum Verhindern einer Verdrehung des Zwischenstückes relativ zur Montageeinheit. Der Federbolzen ist hierbei an der dem Zwischenstück gegenüberliegenden Seite der Montageeinheit hinter der Bohrung der Montageeinheit angeordnet. Der Federbolzen umfasst einen Bolzen, der über eine Feder in einer Sicherungsstellung vorgespannt ist. In dieser Sicherungsstellung ragt der Bolzen durch beide sich überdeckende Aussparung, also sowohl die Aussparung der Montageeinheit und die Aussparung des Zwischenstückes hindurch, so dass die eingestellte Ausrichtung zwischen Montageeinheit und Zwischenstück, also entweder die erste oder die zweite Montageposition, erhalten bleibt und kein unbeabsichtigtes Verdrehen erfolgen kann. Der Bolzen ist manuell entgegen der Federkraft in eine Verdrehstellung bewegbar, in der er zumindest in die Aussparung des Zwischenstückes nicht mehr hineinragt, so dass das Zwischenstück zwischen der ersten und der zweiten Montageposition manuell verdreht werden kann.

Durch die vorrichtungsseitige Befestigungseinheit wird eine schnelle und unkomplizierte Veränderung der Höhe der Vorrichtung relativ zum Operationstisch erreicht, so dass die Lagerung bestmöglich an die individuelle Anatomie des Patienten angepasst werden kann. Ferner kann hierüber, wenn die höchstmögliche Stellung eingenommen ist, ein C-Bogen eines Röntgengerätes auch bei der tiefsten Stellung des Operationstisches noch unter die Vorrichtung bewegt werden, so dass ein Röntgenbild aufgenommen werden kann.

Des Weiteren wird durch die Verstellung ein möglichst kompakter niedriger Gesamtaufbau erreicht.

Die Vorrichtung weist insbesondere zwei Schienen auf, an denen die vorrichtungsseitige Befestigungseinheiten angebracht werden können.

Ein weiterer Aspekt der Erfindung betrifft eine Befestigungsanordnung zur Befestigung einer Vorrichtung zum Lagern eines zu röntgenden Patienten während einer Operation am Operationstisch, die mindestens eine operationstischseitige Befestigungseinheit der zuvor beschriebenen Art und ebenfalls mindestens eine vorrichtungsseitige Befestigungseinheit der zuvor beschriebenen Art umfasst. Bei der Kombination dieser beiden aufeinander abgestimmten Befestigungseinheiten wird eine Verstellbarkeit bei einfacher und sicherer Bedienbarkeit erreicht.

Ein weiterer Aspekt der Erfindung betrifft eine Anordnung, die einen Operationstisch, eine Vorrichtung zum Lagern eines zu röntgenden Patienten während einer Operation und jeweils zwei vorrichtungsseitige und zwei operationstischseitige Befestigungseinheiten umfasst. Die Vorrichtung zum Lagern des Patienten hat zwei Schienen, wobei an jeder der Schienen jeweils eine vorrichtungsseitige Befestigungseinheit angebracht ist. An dem Operationstisch sind die beiden operationstischseitigen Befestigungseinheiten angebracht. Die an der ersten Schiene angeordnete vorrichtungsseitige Befestigungseinheit ist mit einer ersten der operationstischseitigen Befestigungseinheit im Eingriff, die an der zweiten Schiene der Vorrichtung angebrachte vorrichtungsseitige Befestigungseinheit entsprechend mit der zweiten operationstischseitigen Befestigungseinheit.

Da die Schienen soweit jeweils ausschließlich über die jeweiligen Befestigungseinheiten an dem Operationstisch befestigt sind und beide operationstischseitigen Befestigungseinheiten einzeln gelöst werden müssen, ist es nicht notwendig, eine Querverbindung zwischen den Befestigungseinheiten auszubilden. Somit ist in dem zwischen den Stangen angeordneten Bereich möglichst wenig Material angeordnet, so dass wenige Störkonturen beim Röntgen auftreten. Darüber hinaus ermöglicht die einzelne unabhängige Befestigung der beiden Schienen an dem Operationstisch eine möglichst große Abwinkelung der operationstischseitigen Befestigungseinheiten relativ zum Operationstisch, so dass eine entsprechende Höhenverstellung umgesetzt werden kann.

Die beiden operationstischseitigen Befestigungseinheiten sind insbesondere nicht direkt über eine Verbindungseinheit verbunden. Ebenso sind auch die beiden vorrichtungsseitigen Befestigungseinheiten nicht direkt über eine Verbindungseinheit miteinander verbunden. Unter einem direkten Verbinden wird insbesondere verstanden, dass ein Element die beiden Befestigungseinheiten miteinander verbindet, insbesondere dass diese einstückig ausgebildet sind.

Die operationstischseitigen Befestigungseinheiten sind insbesondere analog zu den zuvor beschriebenen operationstischseitigen Befestigungseinheiten gemäß dem zuerst genannten Aspekt der Erfindung ausgebildet.

Ebenso sind die vorrichtungsseitigen Befestigungseinheiten vorzugsweise entsprechend den zuvor beschriebenen vorrichtungsseitigen Befestigungseinheiten gemäß dem zweiten genannten Aspekt der Erfindung ausgebildet. Die Verwendung der zuvor beschriebenen Befestigungseinheiten hat den Vorteil, dass eine Querverbindung zwischen der Befestigungseinheit als Bedienelement entfallen kann und weniger Störkonturen im Bereich der Schnittstellenlagerung sind, so dass der röntgenbare Bereich vergrößert wird. Insbesondere wird der Anteil an Metallstrukturen verringert, welche sich besonders stark in Röntgenbildern abzeichnen.

Darüber hinaus ermöglicht die Befestigung über die zuvor beschriebenen Bedieneinheiten, dass eine möglichst große Abwinkelung zwischen der Vorrichtung und dem Operationstisch möglich ist. Somit kann eine erhöhte Dehnung und/oder Spreizung der Wirbelkörper und ein besserer Zugang zum betroffenen Gewebe erreicht werden. Insbesondere ist eine Patientenlagerung in der sogenannten Z-Position möglich. Darüber hinaus ist eine Einstellung des Winkels während der Operation bei Beibehalten der Sterilität möglich. Ferner besteht mehr Platz, um den C-Bogen eines Röntgengerätes zu platzieren, wodurch Kollisionen vermieden werden.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Fig. 1: eine schematische, perspektivische Darstellung einer Anordnung zum Lagern eines Patienten während einer Rückenoperation;
- Fig. 2: eine Draufsicht einer Vorrichtung zum Lagern eines Teiles des Patienten der Anordnung nach Fig. 1;
- Fig. 3: eine Seitenansicht von Patientenlagereinheiten der Vorrichtung nach Fig. 2;
- Fig. 4: eine schematische, perspektivische Darstellung der Vorrichtung nach Fig. 2 in einem demontierten Zustand;
- Fig. 5: eine schematische, perspektivische Darstellung einer Befestigungsanordnung zur Befestigung der Vorrichtung nach den Figuren 2 und 4 an einem Operationstisch;
- Fig. 6: eine schematische, perspektivische Darstellung einer operationstischseitigen Befestigungseinheit der Befestigungsanordnung nach Fig. 5;
- Fig. 7: eine Seitenansicht der Befestigungseinheit nach Fig. 6;
- Fig. 8: eine schematische, perspektivische Darstellung der innen liegenden Bauteile der Befestigungseinheit der Fig. 6 und 7;
- Fig. 9: eine Seitenansicht der Befestigungseinheit der Fig. 6 bis 8 in einem weiteren Betriebszustand;
- Fig. 10: einen Ausschnitt der Befestigungseinheit nach den Fig. 6 bis 9;
- Fig. 11: eine Seitenansicht der Befestigungseinheit nach den Fig. 6 bis 9 mit Blick auf die der Seitenansicht der Fig. 7 und 9 entgegengesetzte Seite;
- Fig. 12: einen Ausschnitt der Befestigungsanordnung nach den Fig. 7 bis 11 bei einer geneigten Anordnung;
- Fig. 13: einen Ausschnitt der Befestigungseinheit nach den Fig. 6 bis 11 bei einer weiteren geneigten Anordnung der Befestigungseinheit;
- Fig. 14: eine schematische, perspektivische Darstellung der vorrichtungsseitigen Befestigungseinheit der Befestigungsanordnung nach Fig. 5;
- Fig. 15: eine weitere schematische, perspektivische Darstellung der Befestigungseinheit nach Fig. 14;
- Fig. 16: eine schematische, perspektivische Darstellung zweier Befestigungseinheiten nach den Fig. 14 und 15 und zweier Schienen in einer ersten Montageposition; und
- Fig. 17: eine schematische Darstellung der Befestigungseinheiten und der Schienen nach Fig. 16 in einer zweiten Montageposition.

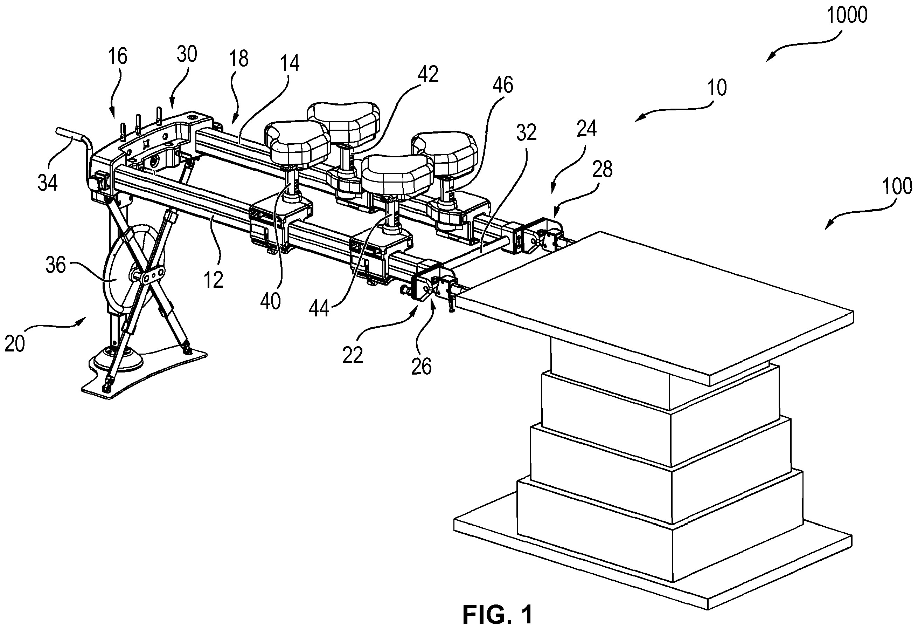

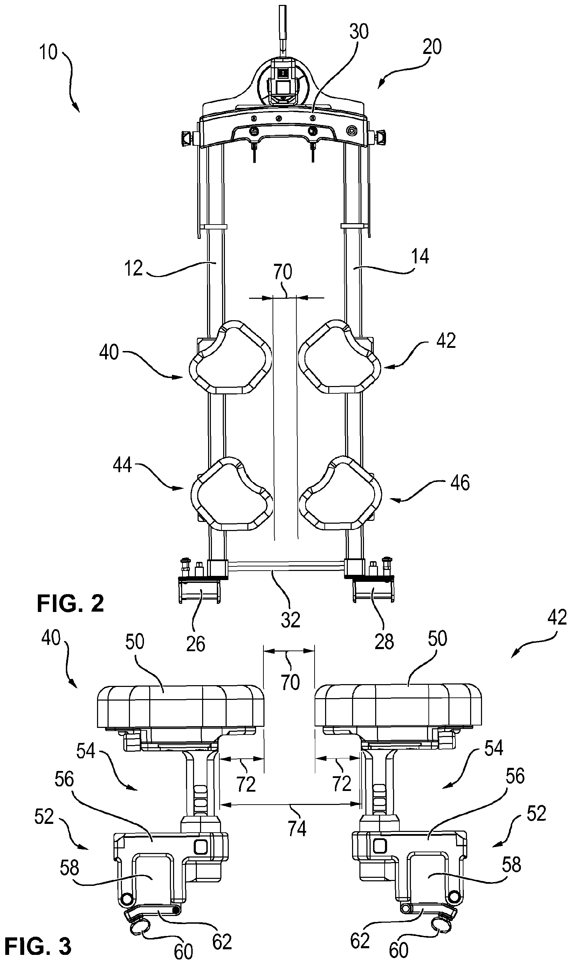

In Fig.1 ist eine schematische, perspektivisch Darstellung einer Anordnung 1000 zum Lagern eines Patienten während einer Operation dargestellt. Die Anordnung umfasst einen Operationstisch 100 sowie eine Vorrichtung 10 zum Lagern des zu röntgenden Körperbereichs des Patienten. Fig.2 zeigt eine Draufsicht auf diese Vorrichtung 10 nach Fig.1.

Die Vorrichtung 10 wird insbesondere verwendet, um Patienten während einer Rückenoperation zu lagern. Bei solchen Rückenoperationen, insbesondere der Wirbelsäule, müssen die Patienten in der Regel während der Operation geröntgt werden, wozu ein C-förmiges Röntgengerät verwendet wird. Insbesondere wird der C-Bogen des Röntgengeräts zur Aufnahme von 3D-Bildern um den Patienten bewegt. Wie im Folgenden noch näher ausgeführt wird, ist die Vorrichtung 10 derart ausgebildet, dass mit ihr auf einfache Weise ein qualitativ hochwertiges Röntgenbild erzeugt werden kann. Ein Patient kann nicht auf einem "normalen" Operationstisch 100 gelagert werden, da ein solcher Operationstisch ein Röntgen mit der notwendigen Qualität und in den erforderlichen Bereichen nicht ermöglicht. Zum einen begrenzt die massive Fußsäule des Operationstisches 100 die mögliche Röntgenlänge und zum anderen umfasst ein normaler Operationstisch 100 zu viele metallhaltige konstruktive Elemente, die das Röntgenbild negativ beeinflussen. Außerdem ist die Patientenlagerfläche des Operationstisches 100 zu breit für die Aufnahme von 3D-Aufnahmen mit Hilfe eines verschwenkbaren C-Bogens.

Die Vorrichtung 10 umfasst zwei Schienen 12, 14, deren erste Enden 16, 18 an einem Ständer 20 gelagert sind. Die den ersten Enden 16, 18 entgegengesetzten zweiten Enden 22, 24 der Schienen 12, 14 können über Befestigungseinheiten 26, 28 an dem Operationstisch 100, insbesondere an Schnittstellen zur Verbindung des Operationstisches 100 mit Beinplatten, befestigt werden.

Der Ständer 20 dient zum einen zum Lagern der Schienen 12, 14 auf dem Boden und zum anderen dazu, um eine vorbestimmten Abstand zwischen den Schienen 12, 14 sicherzustellen, indem der Ständer 20 eine Verbindungseinheit 30 umfasst, über die die beiden ersten Enden 16, 18 der Schienen 12, 14 miteinander verbunden sind.

Ebenso sind die zweiten Enden 22, 24 der Schienen 12, 14 ebenfalls über eine Verbindungseinheit 32 miteinander verbunden, so dass der gewünschte Abstand zwischen den Schienen 12, 14 eingehalten ist. Die Schienen 12, 14 verlaufen parallel zueinander.

Der Ständer 20 ist derart ausgebildet, dass er höhenverstellbar ist, d.h. dass der Abstand der Schienen 12, 14 zum Boden verändert werden kann. Hierzu umfasst der Ständer 20 zum einen eine Handkurbel 34 mit deren Hilfe die Höhe verändert werden kann und zum anderen ein Handrad 36 zum Feststellen und Versteifen des Standfußes des Ständers 20.

Der Operationstisch, an dem die Schienen 12, 14 über die Befestigungseinheiten 26, 28 befestigt sind, ist vorzugsweise mit Hilfe eines Stellantriebs höhenverstellbar, so dass durch entsprechendes Einstellen des Ständers 20 und des Operationstisches die Schienen 12, 14 in einer geeigneten Höhe horizontal angeordnet werden können.

Auf den Schienen 12, 14 sind jeweils zwei Patientenlagereinheiten 40 bis 46 zum Lagern des Patienten angeordnet. In Fig.3 sind zwei dieser Patientenlagereinheiten 40, 42 in einer Seitenansicht dargestellt, wobei zur Vereinfachung der Darstellung die Schienen 12, 14 nicht dargestellt wurden.

Die Patientenlagereinheiten 40 bis 46 umfassen jeweils ein Auflagepad 50, auf dem der Patient aufliegt. Ferner haben die Patientenlagereinheiten 40 bis 46 jeweils eine Befestigungseinheit 52 zur Befestigung der jeweiligen Patientenlagereinheit 40 bis 46 an der jeweiligen Schiene 12, 14 sowie eine Höhenverstelleinheit 54, über die das Auflagepad 50 mit der Befestigungseinheit 52 verbunden ist und über die der Abstand zwischen dem Auflagepad 50 und der Befestigungseinheit 52 eingestellt werden kann.

Die Befestigungseinheit 52 umfasst einen U-förmigen Grundkörper 56, in dessen Aussparung 58 die jeweilige Schiene 12, 14 aufgenommen werden kann. An dem offenen Ende des U-förmigen Grundkörpers ist ein über eine Schraube 60 verriegelbarer Riegel 62 vorgesehen. Zur Montage der Patientenlagereinheit 40 bis 46 wird die Befestigungseinheit 52 beim geöffneten Riegel 62 auf die jeweilige Schiene 12, 14 aufgesetzt, so dass diese in der U-förmigen Aussparung 58 aufgenommen ist. Anschließend wird der Riegel 62 verschlossen und über die Schraube 60 befestigt, so dass eine sichere und dennoch einfache und schnelle Befestigung der Patientenlagereinheit 40 bis 46 an den Schienen 12, 14 möglich ist.

Die Patientenlagereinheiten 40, 42 dienen insbesondere zur Lagerung des Oberkörpers eines Patienten, wohingegen die Patientenlagereinheiten 44, 46 zur Lagerung der Hüfte des Patienten dienen. Der Patient liegt somit mit seinem Kopf in Richtung des Ständers 20, wobei seine Beine auf einem Teil der Patientenlagerfläche des eigentlichen Operationstisches 100 aufliegen. Zur Stützung des Kopfes sind insbesondere noch weitere, nicht dargestellte Patientenlagereinheiten an den Schienen 12, 14 befestigt.

Die Patientenlagereinheiten 40, 44 sind ausschließlich an der ersten Schiene 12, die Patientenlagereinheiten 42, 46 ausschließlich an der zweiten Schiene 14 befestigt. Es bestehen keine Verbindung zwischen den nebeneinander angeordneten Patientenlagereinheiten 40 und 42 sowie 44 und 46.

Die Patientenlagereinheiten 40 bis 46 sind daher unabhängig voneinander einzeln auf den Schienen 12, 14 verschiebbar, so dass zusammen mit der Höhenverstellung der Patientenlagereinheiten 40 bis 46 eine optimale Anpassung an die individuelle Anatomie eines zu operierenden Patienten möglich ist. Insbesondere müssen somit die Patientenlagereinheiten 40 bis 46, anders als dargestellt, nicht immer direkt nebeneinander angeordnet sein. Darüber hinaus können die Auflagepads 50 in unterschiedlichen Höhen angeordnet sein. Ferner kann der Abstand zwischen den Auflagepads 50 von benachbarten Patientenlagereinheiten 40 und 42 bzw. 44 und 46 variiert werden, indem die Auflagepads auf der Höhenverstelleinheit 54 entsprechend quer verschieblich gelagert sind.

Wie den Fig.2 und 3 gut zu entnehmen ist, sind der Abstand zwischen den Schienen 12 und 14 und die Abmessungen der Patientenlagereinheiten 40 bis 46 derart gewählt, dass zwischen den einander zugewandten Seiten an verschiedenen Schienen 12, 14 befestigter Auflagepads 50 ein Freiraum ausgebildet ist. Über diesen Abstand der Auflagepads 50 sowie durch das Nichtverbinden der nebeneinander angeordneten Patientenauflageeinheiten 40, 42 bzw. 44, 46 wird erreicht, dass ein materialfreier Röntgenbereich ausgebildet ist, der in Fig.2 durch ein Rechteck angedeutet und mit dem Bezugszeichen 70 bezeichnet ist. In diesem materialfreien Röntgenbereich 70 werden die Röntgenstrahlen von keinem Material beeinflusst, so dass ein optimales Röntgenbild, insbesondere der bei korrekter Lagerung des Patienten in diesem Bereich angeordneten Wirbelsäule, möglich ist.

Die Auflagepads 50 der Patientenlagereinheiten 40 bis 46 sind insbesondere aus einem röntgenstrahlendurchlässigen Material, beispielsweise einem kohlefaserverstärkten Kunststoff, ausgebildet. Darüber hinaus ist ihre Form in einem an den Röntgenbereich angrenzenden Bereich möglichst homogen und dünn ausgebildet, um Röntgenstrahlenabsorptionsunterschiede, die sich in einem Röntgenbild abbilden würden, zu vermeiden. Somit sind die Auflagepads 50, zumindest in den Bereichen, in denen sie von der Befestigungseinheit 52 und der Höhenverstelleinheit 54 in Richtung des Röntgenbereichs 50 überstehen, für Röntgenstrahlen sehr gut durchlässig, so dass sich, wie in Fig.3 gezeigt, jeweils ein zusätzlicher Röntgenbereich 72 ergibt, in dem ein Röntgenbild mit einer sehr guten Qualität aufgenommen werden kann. Zusammen mit dem Röntgenbereich 70 ergibt sich somit ein erweiterter Röntgenbereich 74, der während der Operation genutzt werden kann.

Die Befestigungseinheit 52 und die Höhenverstelleinheit 54 sind derart ausgebildet, dass sie nur möglichst wenig über die Schiene 12, 14 in Richtung des Röntgenbereiches 70 überstehen, so dass ein möglichst großer Zusatzröntgenbereich 72 ausgebildet ist und somit der erweiterte Röntgenbereich 74 möglichst groß ist.

Die zuvor beschriebene Vorrichtung 10 ermöglicht somit zum einen eine möglichst große Flexibilität bei der Anpassung an die individuelle Anatomie eines Patienten und zum anderen die Aufnahme möglichst hochqualitativer Röntgenbilder.

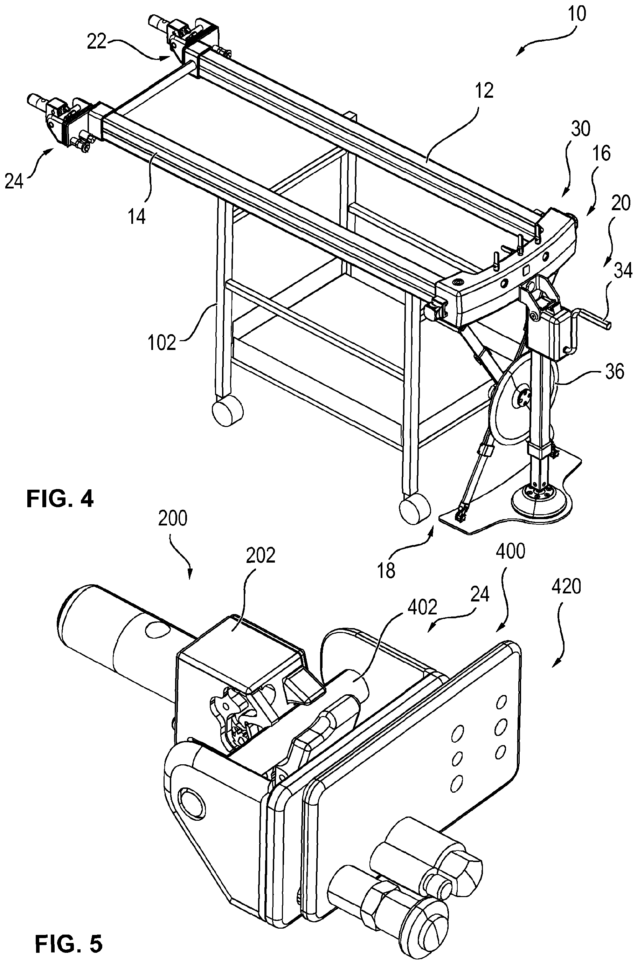

In Fig. 4 ist eine schematische, perspektivische Darstellung der Vorrichtung 10 nach Fig. 2 gezeigt, wobei hierbei ein demontierter Zustand dargestellt ist, in dem die Vorrichtung 10 nicht mit dem Operationstisch 100 verbunden ist. Stattdessen sind die Schienen 12, 14 auf einem verfahrbaren Wagen 102 gelagert, so dass die Vorrichtung 10 somit auf einfache Weise an ihrem Bestimmungsort gebracht werden kann.

In Fig. 5 ist einer schematisch, perspektivische Darstellung der Befestigungsanordnung 26 dargestellt, über die die erste Schiene 12 mit dem Operationstisch 100 verbunden ist. Die zweite Befestigungseinheit 28 ist insbesondere analog aufgebaut, so dass die nachstehenden Ausführungen für diese entsprechend gelten.

Die Befestigungsanordnung 26 umfasst eine operationstischseitige Befestigungseinheit 200, die an Schnittstellen zur Aufnahme von Beinplatten des Operationstisches 100 befestigt werden kann, sowie eine vorrichtungsseitige Befestigungseinheit 400, die an den zweiten Enden 22, 24 der Schienen 12, 14 befestigt werden kann, weshalb die vorrichtungsseitige Befestigungseinheit 400 im Rahmen dieses Ausführungsbeispiels als schienenseitige Befestigungseinheit 400 bezeichnet wird. Über die Befestigungsanordnung 26 kann die jeweilige Schiene 12, 14 auf einfache Weise lösbar an dem Operationstisch 100 befestigt werden.

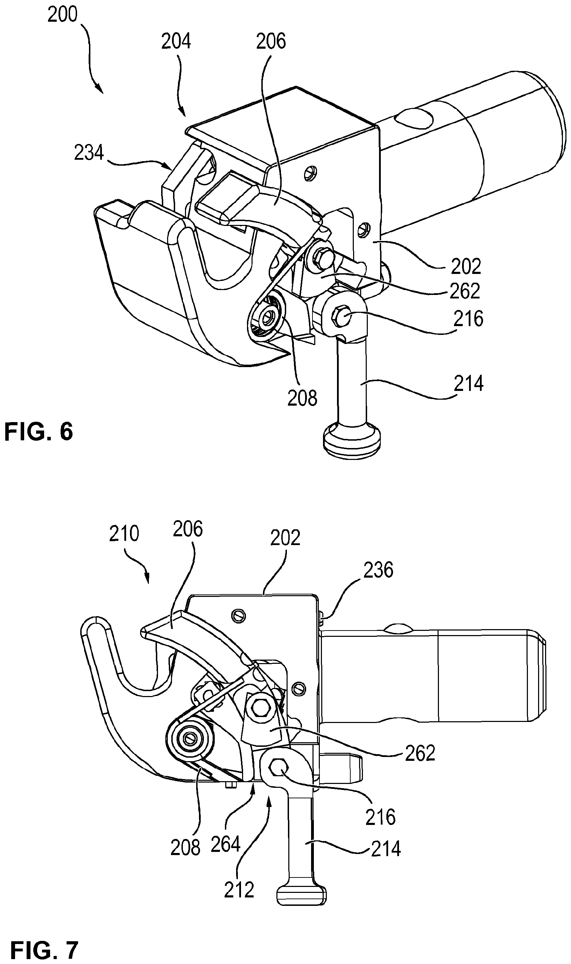

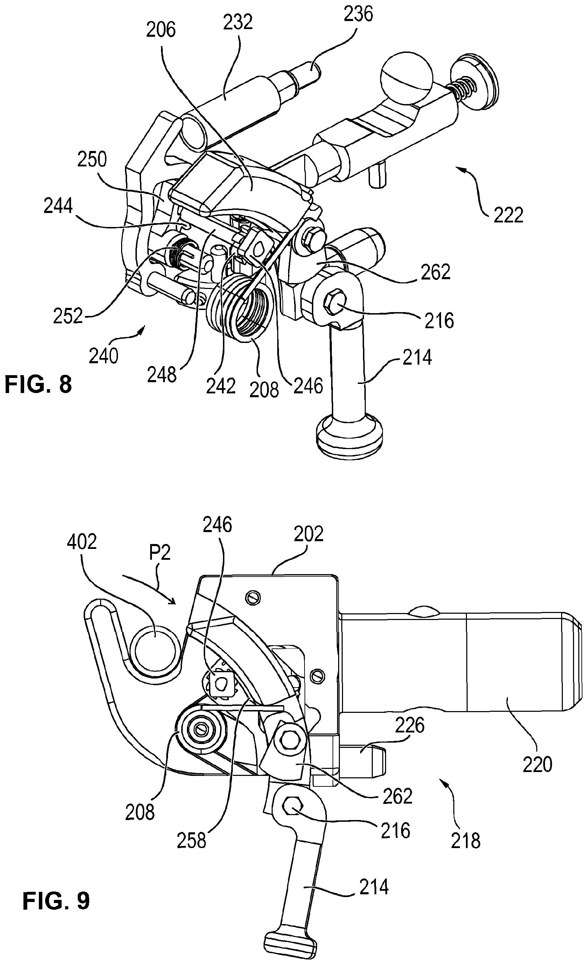

In den Fig. 6 bis 13 ist jeweils die operationstischseitige Befestigungseinheit 200 dargestellt, die in der Beschreibung dieser Figuren der Einfachheit halber nur als die Befestigungseinheit 200 bezeichnet wird. Hierbei ist bei allen Figuren, wie auch bei Fig. 5, auf die Darstellung von seitlichen Abdeckungen des Gehäuses verzichtet worden, um die innenliegenden Bauteile darzustellen. In Fig. 8 wurde zusätzlich auf die Darstellung eines Grundkörpers 202 der Befestigungseinheit 200 verzichtet, um die innenliegenden Bauteile noch besser sichtbar zu machen.

In den verschiedenen Figuren sind teilweise verschiedene Betriebszustände dargestellt, die im Folgenden nach der Beschreibung des Aufbaus im Zusammenhang mit der Beschreibung der Funktion näher erläutert werden.

Die Befestigungseinheit 200 umfasst den Grundkörper 202, die zur Lagerung der weiteren später noch näher beschriebenen Baueinheiten dient. Der Grundkörper 202 weist eine V-förmige Aufnahme 204 auf, in der ein als Stange 402 ausgebildetes Koppelelement der schienenseitigen Befestigungseinheit 400 aufgenommen werden kann, um hierüber die Schiene 12, 14 entsprechend zu befestigen.

Die Befestigungseinheit 200 umfasst ferner einen Riegel 206, der zwischen einer in den Figuren 6, 7 und 11 dargestellten verriegelten Position und einer in Fig. 9 gezeigten entriegelten Position relativ zum Grundkörper 204 bewegbar ist. Der Riegel 206 ist hierbei über eine Drehfeder 208 in der verriegelten Position vorgespannt und kann entgegen der Rückstellkraft dieser Drehfeder 208 in die entriegelte Position bewegt werden.

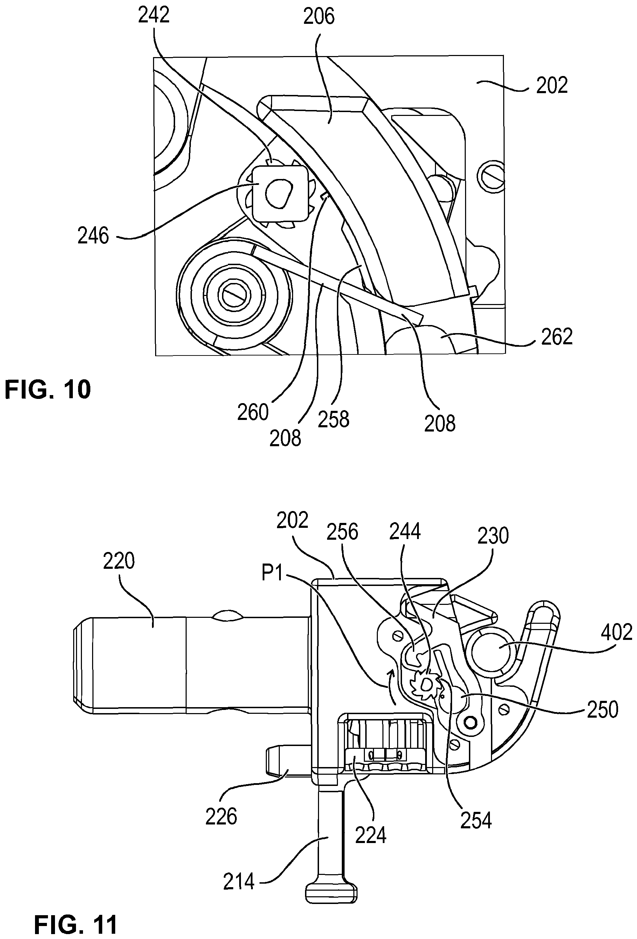

In der verriegelten Position ragt ein erstes Ende 210 des Riegels 206 derart in die Aussparung 204 hinein, dass, wie in Fig. 11 dargestellt, die Stange 402 nicht aus der Aussparung 204 entnommen werden kann, sofern nicht zuvor der Riegel 206 in die entriegelte Position bewegt wurde.

Zum Bewegen des Riegels 206 ist an dem dem ersten Ende entgegengesetzten zweiten Ende 212 ein Zughebel 214 als Betätigungselement angeordnet. Dieser ist insbesondere relativ zum Riegel drehbar über einen Stift und eine Mutter 216 befestigt, so dass der Zughebel 214 relativ zum Grundkörper 202 abgeklappt werden kann, und somit möglichst wenig im Arbeitsbereich stört. Auch die Anordnung an der Unterseite der Befestigungseinheit 200 bewirkt, dass der Hebel 214 nicht unbeabsichtigt betätigt werden kann, möglichst wenig stört und ergonomisch günstig bedienbar ist.

Ferner umfasst die Befestigungseinheit 200 eine Verbindungseinheit 218, über die die Befestigungseinheit 200 an dem Operationstisch 100 befestigbar ist. Hierbei wird die Stange 220 in einer entsprechenden Aussparung des Operationstisches 100 eingeführt und rastet über einen Rastmechanismus 222 in der Schnittstelle des Operationstisches 100 ein, so dass die Befestigungseinheit 200 sich nicht unbeabsichtigt lösen kann. An der Unterseite der Befestigungseinheit 200 ist ein Knopf 224 angeordnet, mit dem die Rastverbindung wieder gelöst werden kann, so dass die Befestigungseinheit 200 vom Operationstisch 100 entfernt werden kann.

Ferner umfasst die Verbindungseinheit 218 einen Stift 226, der in eine entsprechende Aussparung des Operationstisches eingreift und somit ein Verdrehen um die Längsachse der Stange 220 verhindert.

Darüber hinaus umfasst die Befestigungseinheit 200 einen Taster 230, der über ein elastisches Element 232 in einer in den Figuren 6 und 7 gezeigten ersten Position vorgespannt ist, in der er in die Aussparung 204 hineinragt. Wird eine Stange 402 in die Aussparung 204 eingeführt, so kontaktiert diese den Taster 230 an seiner abgeschrägten Fläche 234 und bewegt darüber den Taster 230 von der ersten Position in eine in den Figuren 9 und 11 dargestellte zweite Position, in der der Taster 230 nicht mehr in die Aufnahme 204 hineinragt. Solange wie die Stange 402 in der Aufnahme 204 aufgenommen ist, hält diese den Taster 230 in der zweiten Position. Beim Bewegen von der ersten in die zweite Position wird ein Silikonblock 236 an der dem Operationstisch 100 zugewandten Seite der Befestigungseinheit 200 aus dieser hinaus bewegt, so dass eventuell vorhandenes Spiel zwischen dem Operationstisch 100 und der Befestigungseinheit 200 minimiert wird.

Darüber hinaus weist die Befestigungseinheit 200 einen Gesperremechanismus 240 auf, über den der Riegel 206 unter bestimmten Betriebsvoraussetzungen in der entriegelten Position gehalten wird.

Dieser Gesperremechanismus weist ein erstes Klinkenrad 242, ein zweites Klinkenrad 244, eine Vierkantscheibe 246, eine Welle 248 sowie ein Sperrelement 250 auf. Die Klinkenräder 244 sowie die Vierkantscheibe 246 sind drehfest auf der Welle 248 angeordnet, so dass die hieraus gebildete Einheit nur zusammen gedreht werden kann. Das Sperrelement 250 ist über eine Drehfeder 252 in einer Sperrposition vorgespannt, die in Fig. 11 gezeigt ist. In dieser Sperrposition greift ein Vorsprung 254 des Sperrelementes 250 in eine Klinke des zweiten Klinkenrades 244 ein und verhindert dadurch eine Drehung des zweiten Klinkenrades 244 in eine erste Drehrichtung P1. Die Form des Klinkenrades 244 und die Form des Sperrelementes 250 sind derart aufeinander abgestimmt, dass eine Drehung entgegen der ersten Richtung P1 abhängig der Position des Sperrelementes 250 möglich ist.

Ist der Taster 230 ist der ersten Position angeordnet, so hält er über den Kontakt zwischen einer Nase 256 und dem Element 250 dieses in einer gelösten Position, in der es nicht mehr in Eingriff mit dem zweiten Klinkenrad 244 steht und somit das zweite Klinkenrad 244 und damit die Welle 248 und die anderen auf ihm gelagerten Bauteile auch in die erste Drehrichtung P1 drehbar sind.

Da, wie zuvor beschrieben, der Taster 230 in der ersten Position vorgespannt ist, ist das Sperrelement 250, solange wie keine Stange 402 in der Aufnahme 204 aufgenommen ist, in der gelösten Position angeordnet. Ist dagegen eine Stange 402 in der Aufnahme 204 aufgenommen, so ist der Taster 230 in die zweite Position bewegt, so dass das Sperrelement 250 in der Sperrposition angeordnet ist.

Somit ist, wenn keine Stange 402 in der Aufnahme 204 aufgenommen ist, die Welle 248 in die erste Richtung drehbar, so dass der Gesperremechanismus 240 gelöst ist. Ist dagegen eine Stange 402 in der Aufnahme 204 aufgenommen, sperrt das Sperrelement 250 eine Drehung der Welle 248 in die erste Richtung P1, so dass der Gesperremechanismus 240 gesperrt ist.

Je nach Drehstellung der Welle 248 ist die Vierkantscheibe 246 entweder in einer Sperrwinkelstellung oder einer Entsperrwinkelstellung angeordnet. Die Entsperrwinkelstellung ist hierbei in Fig. 7, die Sperrwinkelstellung in Fig. 9 und 10 dargestellt. Hierbei sind die Form des ersten Klinkenrades 242 und der Vierkantscheibe 246 derart aufeinander abgestimmt, dass, wenn das erste Klinkenrad 242 um eine Klinke weiter entgegen der ersten Drehrichtung P1 gedreht wird, die Stellung der Vierkantscheibe 248 immer zwischen der Sperrwinkelstellung und der Entsperrwinkelstellung wechselt.

An dem Riegel 206 ist ein Vorsprung 258 ausgebildet, der, wenn die Vierkantscheibe 246 in der Sperrwinkelstellung angeordnet ist, mit dieser in Eingriff steht. Sofern der Gesperremechanismus 240 gesperrt ist, also eine Drehung der Welle 248 entgegen der Richtung P1 verhindert ist, verhindert der Kontakt zwischen dem Vorsprung 248 und der Vierkantscheibe 246, dass sich der Riegel 206 von der entriegelten in die verriegelte Position bewegt, wenn der Riegel 206 zuvor über den Zughebel 214 über die entriegelte Position heraus in Richtung des Pfeiles P2 weiter bewegt wurde. Dieser Zustand ist beispielsweise in Fig. 9 gezeigt.

Ist dagegen der Gesperremechanismus 240 gelöst, so würde die Welle 248 über die Spannung des Riegels 206 in die erste Drehrichtung gedreht, so dass sich der Riegel 206 automatisch in die verriegelte Position bewegen würde.

Ferner ist in einer Aussparung des Riegels 206 ein Kontaktelement 260 federnd gelagert. Wenn der Riegel 206 von der verriegelten in die entriegelte Position bzw. über diese entriegelte Position hinaus bewegt wird, also anders gesagt, wenn der Zughebel 214 vollständig betätigt wird, so wird das Klinkenrad 242 um eine Klinke weiter entgegen der ersten Richtung P1 gedreht, so dass die Vierkantscheibe 246 von der Entsperrwinkelstellung in die Sperrwinkelstellung gedreht wird. Anschließend bewegt sich der Riegel 206 von der über die verriegelte Position heraus bewegten Stellung (Fig. 10) wieder zurück in Richtung der verriegelten Position, wobei das Kontaktelement 260 dann über den Kontakt zu der abgeschrägten Seite der Klinken des ersten Klinkenrades 242 in den Riegel 206 hinein bewegt wird, so dass selbst wenn der Gesperremechanismus 240 gesperrt ist, sich der Riegel 206 solange in Richtung der verriegelten Position bewegen kann, bis der Eingriff zwischen dem Vorsprung 258 und der Vierkantscheibe 246 besteht und der Riegel 206 entsprechend in der entriegelten Position, wie in Fig. 9 gezeigt, angeordnet ist.

Solange noch keine Stange 402 in der Aufnahme 204 aufgenommen ist, ist der Taster 230 in der ersten Position angeordnet, so dass der Gesperremechanismus 240 gelöst ist. Der Riegel 206 ist dann in der verriegelten Position angeordnet. Selbst wenn der Zughebel 214 betätigt wird, würde sich der Riegel 206 wieder zurück in die verriegelte Position bewegen, da aufgrund des gelösten Gesperremechanismus 240 die Welle 248 in die erste Richtung P1 drehbar ist und so unabhängig von ihrer Stellung des Riegels 206 nicht in der entriegelten Position halten würde.

Wird eine Stange 402 in die Aufnahme 204 hineingedrückt, so wird zum Einen der Taster 230 von der ersten in die zweite Position bewegt, so dass der Gesperremechanismus 240 gesperrt wird und ein Drehen in die erste Richtung P1 von nun an unterbunden wird, solange wie die Stange 402 in der Aufnahme 204 angeordnet ist. Zum anderen wird beim Einführen der Stange 402 durch diese der Riegel 206 aus der verriegelten Position in Richtung der entriegelten Position bewegt, aber nicht so weit, dass der Vorsprung 58 hinter die Vierkantscheibe 246 bewegt wird, also nicht so weit, dass der Riegel 206 in der entriegelten Position angeordnet ist. Somit wird erreicht, dass sich der Riegel 206 nach dem Vorbeiführen der Stange 402 wieder automatisch in die entriegelte Position zurück bewegt.

Soll die Stange 402 aus der Aufnahme 204 entnommen werden, so muss zunächst der Riegel 206 von der verriegelten Position in die entriegelte Position bewegt werden. Bei Betätigung des Zughebels 214 wird der Riegel 206 über die Entriegelungsposition hinaus bewegt, wobei durch den Kontakt über das Kontaktelement 260 mit den Klinken des ersten Klinkenrades 242 das erste Klinkenrad 242 um eine Klinke weiter entgegen der Richtung P1 gedreht wird. Hierüber wird die zuvor in der Entsprerrwinkelstellung angeordnete Vierkantscheibe 246 in die Sperrwinkelstellung bewegt. Nachdem der Zughebel 214 losgelassen wurde, bewegt die Drehfeder 208 den Riegel 206 wieder so weit zurück, bis der Vorsprung 258 mit der Vierkantscheibe 246 in Eingriff ist. Da der Gesperremechanismus 240, wie zuvor beschrieben, gesperrt ist, kann sich die Welle 248 nicht in die erste Richtung P1 drehen, so dass die Vierkantscheibe 246 ihre Ausrichtung nicht verändern kann und somit der Riegel 206 in der entriegelten Position gehalten wird, ohne dass hierfür der Zughebel 214 festgehalten werden muss. Wird die Stange 402 aus der Aufnahme 204 entnommen, so bewegt sich der Taster 230 wieder von der zweiten in die erste Position zurück, wodurch der Gesperremechanismus 240 gelöst wird. Somit kann sich die Welle 248 in die erste Richtung P1 drehen, wodurch auch die Vierkantscheibe 246 wieder von der Sperr- in die Entsperrwinkelstellung gedreht wird, so dass diese den Riegel 206 nicht mehr zurückhält und der Riegel 206 sich automatisch wieder in die verriegelte Position bewegt.

Soll dagegen nach der einmalige Betätigung des Zughebels 214 der durch die Vierkantscheibe 246 in der entriegelten Position gehaltene Riegel 206 ohne Entnahme der Stange 402 wieder verriegelt werden, beispielsweise weil der Zughebel 214 unbeabsichtigt betätigt wurde, muss die Bedienperson nur erneut an dem Zughebel 214 ziehen. Hierdurch wird der Riegel 206 aus der entriegelten Position heraus weiter in den Grundkörper 202 in Richtung des Pfeiles P2 hinein bewegt, wodurch erneut das Kontaktelement 260 das erste Klinkenrad 242 um eine Klinke weiter bewegt. Somit wird die Vierkantscheibe 246 wieder von der Sperrwinkelstellung in die Entsperrwinkelstellung bewegt, so dass nach dem Loslassen des Zughebels 214 der Vorsprung 258 sich an der Vierkantscheibe 246 vorbei bewegen kann, und somit der Riegel 206 sich wieder automatisch in die verriegelte Position bewegt.

Über den zuvor beschriebenen Verriegelungsmechanismus wird eine einfache und sichere Verriegelung erreicht, die wenig anfällig für Bedienfehler ist und ein zuverlässiges sicheres Verriegeln, sofern eine Stange 402 aufgenommen ist, ermöglicht. Insbesondere werden auch die notwendigen Bedienschritte minimiert.

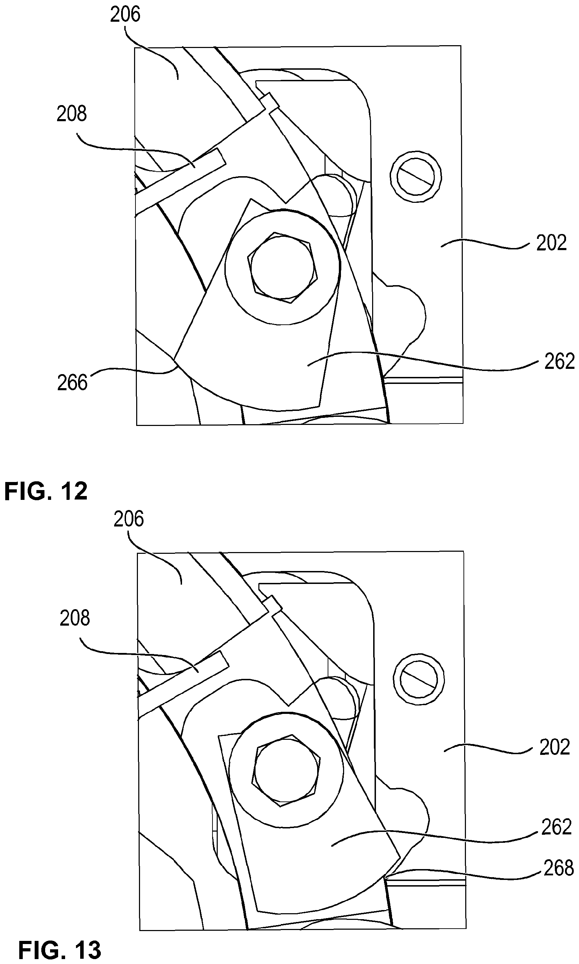

An dem Riegel 206 ist ferner ein Schwerkraftpendel 262 drehbar gelagert, welches, wenn die Befestigungseinheit 200 horizontal angeordnet ist, also wie beispielsweise in Fig. 5 gezeigt, derart ausgerichtet ist, dass es sich bei Betätigen des Zughebels 214 in eine Öffnung 264 bewegt und somit nicht die Bewegung des Riegels 206 stört. Ist die Befestigungseinheit 200 dagegen um einen Winkel, der größer als ein vorbestimmter Grenzwinkel ist, aus der Horizontalen hinaus bewegt, wie in den Fig. 12 und 13 gezeigt ist, wobei Fig. 12 eine Kippung nach vorne und Fig. 13 eine Kippung nach hinten, also in Richtung des OP-Tisches zeigt, so verhindert das Schwerkraftpendel 262 eine Betätigung des Zughebels 214, da es mit Kanten 266, 268 des Grundkörpers in Eingriff steht und somit ein Bewegen des Riegels 206 unterbindet.

Durch dieses Schwerkraftpendel 262 wird somit sichergestellt, dass eine Entriegelung des Riegels 206 nur bei in etwa horizontaler Ausrichtung erfolgen kann, so dass, beispielsweise wenn die Befestigungseinheit 200 nach vorne, also vom Operationstisch 100 weg, geneigt ist, die Stange 402 nicht durch ein unbeabsichtigtes Lösen der Entriegelung aus der Aufnahme 204 hinausrutschen kann.

Alternativ können statt eines Gesperremechanismus auch andere Ausführungsformen eines Flip-Flop-Mechanismus verwendet werden.

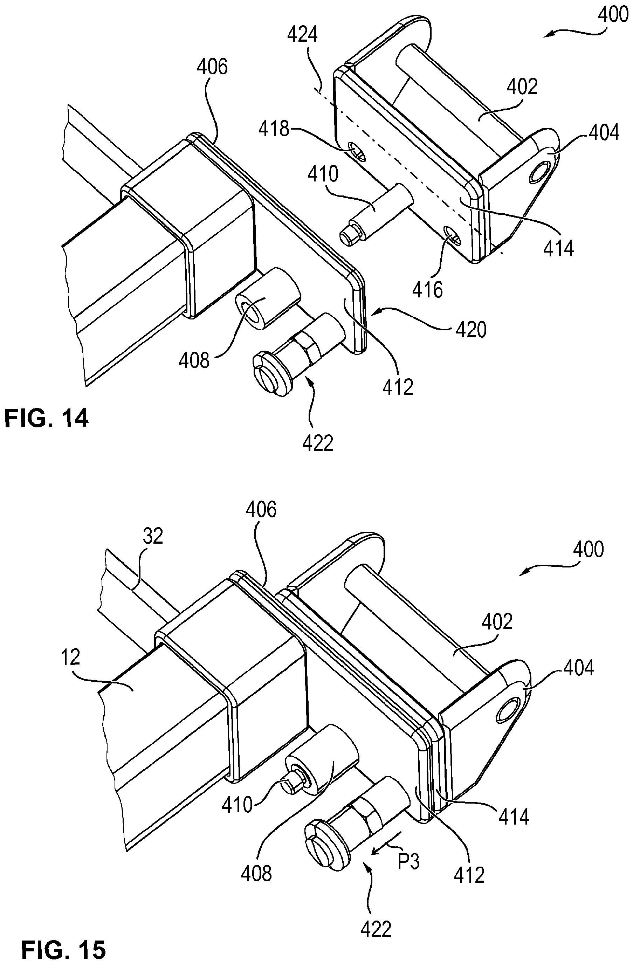

In den Figuren 14 und 15 ist jeweils eine schematisch, perspektivische Darstellung der schienenseitigen Befestigungseinheit 400 nach Fig. 5 gezeigt. Die schienenseitige Befestigungseinheit 400 umfasst ein an den Schienen 12, 14, insbesondere dauerhaft befestigbare Montageeinheit 406 sowie ein Zwischenstück 404, welches die Stange 402 umfasst. In Figur 14 ist das Zwischenstück, welches normalerweise unverlierbar an der Montageeinheit 406 befestigt ist, getrennt von dieser dargestellt, um den Befestigungsmechanismus darzustellen.

Die Montageeinheit 406 umfasst eine Bolzenaufnahme 408, in der ein Bolzen 410 des Zwischenstückes 404 gelagert ist, so dass das Zwischenstück 404 relativ zur Montageeinheit 406 verdrehbar ist, sofern dies nicht über eine Verdrehsicherung unterbunden wird. An dem aus der Bolzenaufnahme 408 herausragendem Ende des Bolzens 410 ist insbesondere eine Sicherung angebracht, die ein Entfernen des Zwischenstücks 404 von der Montageeinheit 406 unterbindet.

Die Montageeinheit 406 und das Zwischenstück 404 umfassen jeweils eine Platte 412, 414, die bei einer möglichen Ausführungsform aneinander anliegen und gegeneinander verdreht werden können. Alternativ kann zwischen den Platen 412, 414 auch ein vorbestimmter Abstand sein, so dass sich diese nicht kontaktieren.

Die Platte 414 des Zwischenstücks 404 weist zwei Bohrungen 416, 418 auf, die im gleichen Abstand zum Bolzen 410 an gegenüberliegenden Seiten des Bolzens 410 angeordnet sind. Insbesondere verläuft eine die beiden Bohrungen 416, 418 miteinander verbindende Linie durch die Längsachse des Bolzens 410, wobei diese Längsachse die die Bohrungen 416, 418 verbindende Linie in der Mitte schneidet. Die Bohrungen 416, 418 und der Bolzen 410 sind bezogen auf eine Mittelachse 424 Platte 410 in eine erste Richtung versetzt angeordnet. Die Stange 402 dagegen ist ebenfalls versetzt zur Mittelachse 424, allerdings in eine der ersten Richtung entgegengesetzte zweite Richtung angeordnet. Bezogen auf die in Fig. 14 gezeigte Ausrichtung sind somit die Bohrungen 416, 418 und der Bolzen 420 unterhalb der Mittellinie 424, die Stange dagegen oberhalb der Mittellinie 424 angeordnet.

Alternativ können anstelle von Bohrungen 410 bis 420 auch andere Aussparungen, z.B. Löcher, vorgesehen sein. Beispielsweise können die Aussparungen der Platte 414 des Zwischenstücks 414 auch nicht durchgängig sein.

Auch die Platte 412 weist eine Bohrung 420 auf, die in Fig. 14 und 15 durch einen Federbolzen 422 verdeckt ist, der an der dem Zwischenstück 404 abgewandten Seite der Montageeinheit 406 angeordnet ist. Die Bohrung 420 hat den gleichen Abstand zur Bolzenaufnahme 408 wie die Bohrungen 416, 418 zum Bolzen 410.

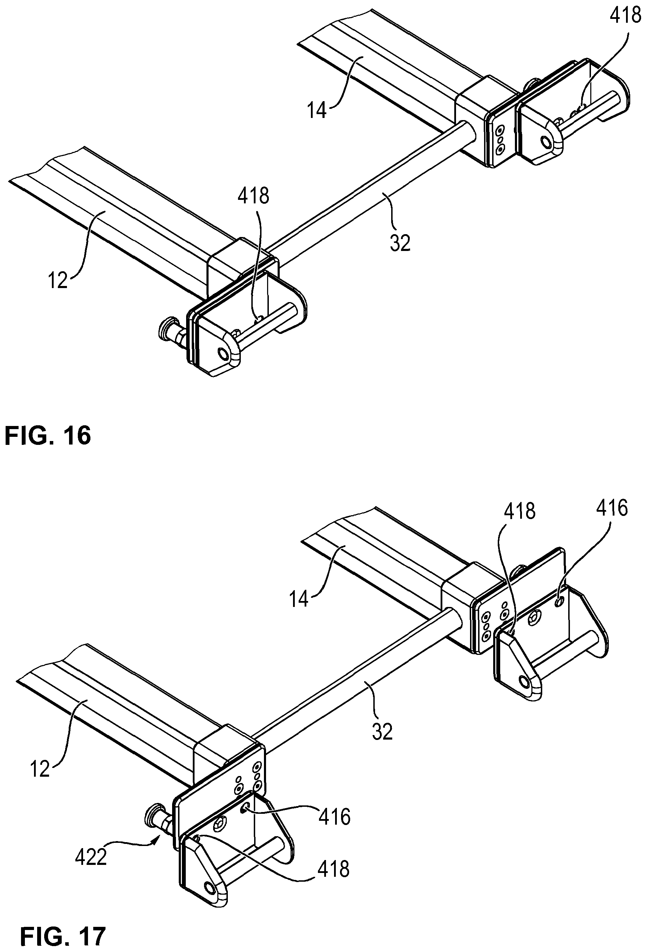

Das Zwischenstück 404 kann in zwei verschiedenen Ausrichtungen relativ zur Montageeinheit 406 an diese arretiert werden. In Fig. 16 ist hierbei eine erste Ausrichtung, in Fig. 17 die zweite Ausrichtung dargestellt.

Bei der in Fig. 16 gezeigten ersten Ausrichtung ist das Zwischenstück 404 derart gedreht, dass seine Bohrung 416 koaxial zur Bohrung 420 der Montageeinheit 406 gerichtet ist, so dass ein Bolzen des Federbolzens 422 durch diese beiden sich überdeckenden Bohrungen 416, 420 hindurchragt und somit ein Verdrehen des Zwischenstückes 404 relativ zur Montageeinheit 406 unterbindet.

Bei der in Fig. 17 gezeigten zweiten Ausrichtung dagegen ist das Zwischenstück 404 relativ zur ersten Ausrichtung um 180° um die Längsachse des Bolzens 410 gedreht, so dass sich nun die Bohrung 418 und die Bohrung 420 überdecken, also koaxial zueinander angeordnet sind. Entsprechend ragt der Bolzen des Federbolzens 422 durch diese beiden Bohrungen durch und verhindert wiederum ein Verdrehen des Zwischenstücks relativ zur Montageeinheit 406. Über das Drehen des Zwischenstücks 404 relativ zur Montageeinheit 406 und die versetzte Anordnung der Stange 402 relativ zur Drehachse ist die Stange 402 in den beiden Ausrichtungen in einer unterschiedlichen Höhe relativ zu den Schienen 12, 14 angeordnet, so dass bei einer Befestigung an der operationstischseitigen Befestigungseinheit 200 entsprechend eine Höhenverstellung erreicht ist.

Insbesondere kann somit eine Höhenverstellung von etwa 7 cm durch Drehung des Zwischenstücks 404 erreicht werden.

Um die Ausrichtung zu ändern, also das Zwischenstück 404 zu drehen, muss der Bolzen des Federbolzens 422 in Richtung des Pfeiles P3 entgegen der Federkraft des Federbolzens 422 bewegt werden, so dass dieser zumindest in die Bohrung 416 bzw. 418 des Zwischenstückes 404 nicht mehr hineinragt. Somit kann, wenn der Bolzen zurückgezogen ist, das Zwischenstück 404 gedreht werden. Wird der Bolzen des Federbolzens 422 wieder losgelassen, so wird er durch die Feder automatisch wieder entgegen der Richtung P3 bewegt, so dass er nun wieder in die vor dem Loch 420 angeordnete Bohrung 416, 418 des Zwischenstücks 404 hineinragt und in der entsprechenden Ausrichtung das Zwischenstück 404 fixiert.

### Bezugszeichenliste

- 10: Vorrichtung
- 12, 14: Schiene
- 16, 18, 22, 24: Ende
- 20: Ständer
- 30, 32: Befestigungsanordnung
- 34: Kurbel
- 36: Handrad
- 40, 42, 44, 46: Patientenlagereinheit
- 50: Auflagepad
- 52: Befestigungseinheit
- 54: Höhenverstelleinheit
- 56: Grundkörper
- 58: Aussparung
- 60: Schraube
- 62: Platte
- 70: Röntgenbereich
- 72: Zusatzröntgenbereich
- 74: erweiterter Röntgenbereich
- 100: Operationstisch
- 200: operationstischseitige Befestigungseinheit
- 202: Grundkörper
- 204: Aufnahme
- 206: Riegel
- 208: Drehfeder
- 210: Ende
- 212: Ende
- 214: Zughebel
- 216: Mutter
- 218: Verbindungseinheit
- 220: Stange
- 222: Rastmechanismus
- 224: Knopf
- 226: Stift
- 230: Taster
- 232: elastisches Element
- 234: Schräge
- 236: Silikonblock
- 240: Gesperremechanismus
- 242, 244: Klinkenrad
- 246: Vierkantscheibe
- 248: Welle
- 250: Sperrelement
- 252: Drehfeder
- 254: Vorsprung
- 256: Nase
- 258: Vorsprung
- 260: Kontaktelement
- 262: Schwerkraftpendel
- 264: Öffnung
- 266, 268: Kante
- 400: schienenseitige / vorrichtungsseitige Befestigungseinheit
- 402: Stange / Koppelelement
- 404: Zwischenstück
- 406: Montageeinheit
- 408: Bolzenaufnahme
- 410: Bolzen
- 412, 414: Platte
- 416, 418, 420: Bohrung / Aussparung
- 422: Federbolzen
- 424: Mittelachse
- 1000: Anordnung
- P1, P2, P3: Richtung

## Patentansprüche

1. Operationstischseitige Befestigungseinheit zur Befestigung einer Vorrichtung zum Lagern eines zu röntgenden Patienten während einer Operation an einem Operationstisch,
mit einem Grundkörper (202), der eine Aufnahme (204) zur Aufnahme eines Koppelelements (402) einer an der Vorrichtung (12, 14) befestigbaren vorrichtungsseitigen Befestigungseinheit (400) hat,
einem an dem Grundkörper (202) relativ zu diesem bewegbar befestigten Riegel (206), wobei der Riegel (206) in einer verriegelten Position ein Entfernen des Koppelelements (402) aus der Aufnahme (204) verhindert und in einer entriegelten Position ein Entfernen des Koppelelements (402) aus der Aufnahme (204) erlaubt,
einem elastischen Element (208), das den Riegel (206) in der Verriegelungsposition vorspannt,
einem Betätigungselement (214) zum manuellen Bewegen des Riegels (206) von der verriegelten in die entriegelte Position, und
mit einem Gesperremechanismus (240) zum Halten des Riegels (206) in der entriegelten Position, **dadurch gekennzeichnet, dass** der Gesperremechanismus (240) den Riegel (206), wenn ein Koppelelement (402) in der Aufnahme (204) angeordnet ist und der Riegel (206) mit Hilfe des Betätigungselements (214) in die entriegelte Position bewegt wurde, zumindest solange in der entriegelten Position hält, bis das Koppelelement (402) aus der Aufnahme (204) entfernt worden ist oder das Betätigungselement (214) erneut betätigt worden ist.

2. Befestigungseinheit (200) nach Anspruch 1, **dadurch gekennzeichnet, dass** die Aufnahme (204) V-förmig, insbesondere mit einem abgerundeten Bodenbereich, ausgebildet ist.

3. Befestigungseinheit (200) nach Anspruch 1 oder 2, **dadurch gekennzeichnet, dass** das Betätigungselement (214) einen an dem Riegel (206), insbesondere drehbar, befestigten Zughebel (214) umfasst, der derart ausgebildet und gelagert ist, dass er sowohl zum Bewegen des Riegels (206) von der verriegelten in die entriegelte Position als auch zum Lösen des Gesperremechanismus (240), so dass der Riegel (206) bei in der Aufnahme (204) aufgenommenem Koppelelement (402) durch das elastische Element (208) zurück in die verriegelte Position bewegt wird, von dem Grundkörper (202) weg gezogen werden muss.

4. Befestigungseinheit (200) nach einem der vorhergehenden Ansprüche, **dadurch gekennzeichnet, dass** an dem Riegel (206) ein Schwerkraftpendel (262) drehbar befestigt ist, und dass das Schwerkraftpendel (262) eine Betätigung des Betätigungselementes (214) unterbindet, wenn die Befestigungseinheit (200) außerhalb eines vorbestimmten Ausrichtungsbereich relativ zur Horizontale angeordnet ist.

5. Befestigungseinheit (200) nach einem der vorhergehenden Ansprüche, **dadurch gekennzeichnet, dass** das in die Aufnahme (204) ragende Ende (210) des Riegels (206) derart abgeschrägt ist, dass der Riegel (206) beim Einführen des Koppelelements (402) in die Aufnahme (204) durch den Kontakt zum Koppelelement (402) automatisch aus der verriegelten Position in Richtung (P2) der entriegelten Position bewegt wird, aber nicht so weit, dass der Gesperremechanismus (240) den Riegel (206) festhält.

6. Befestigungseinheit (200) nach einem der vorhergehenden Ansprüche, **dadurch gekennzeichnet, dass** der Gesperremechanismus (240) als Flip-Flop-Mechanismus ausgebildet ist.

7. Befestigungseinheit (200) nach einem der vorhergehenden Ansprüche, **dadurch gekennzeichnet, dass** die Befestigungseinheit (200) einen drehbar an dem Grundkörper (200) befestigten Taster (230) umfasst, der mit Hilfe eines weiteren elastischen Elements (232) in einer ersten Position vorgespannt ist, in der er zumindest teilweise in die Aufnahme (204) hineinragt, der in einer zweiten Position relativ zur ersten Position entgegen der Rückstellkraft des weiteren elastischen Elements (232) aus der Aufnahme (204) heraus in den Grundkörper (202) hinein bewegt ist, und dass der Taster (230) derart ausgebildet ist, dass er beim Einführen des Koppelelements (402) in die Aufnahme (204) automatisch von der ersten in die zweite Position bewegt wird und durch das aufgenommene Koppelelement (402) in dieser gehalten ist so lange das Koppelelement (402) in der Aufnahme (204) angeordnet ist.

8. Befestigungseinheit (200) nach Anspruch 7, **dadurch gekennzeichnet, dass** der Gesperremechanismus (240) ein erstes Klinkenrad (242), ein zweites Klinkenrad (244) und eine Vierkantscheibe (246) umfasst, die drehfest auf einer gemeinsamen Welle (248) gelagert sind, dass der Gesperremechanismus (240) ein in einer Sperrposition vorgespanntes Sperrelement (250) umfasst, das in der Sperrposition mit dem zweiten Klinkenrad (244) in Eingriff ist und eine Drehung der Welle (248) in eine erste Richtung (PI) verhindert, und dass das Sperrelement (250) in einer gelösten Position eine Drehung der Welle (248) in die erste Richtung (PI) erlaubt, wobei der Taster (230), wenn er in der ersten Position angeordnet ist, das Sperrelement (250) kontaktiert und dieses entgegen seiner Vorspannung in der gelösten Position hält, und wobei das Sperrelement (250) in der Sperrposition angeordnet ist, wenn der Taster (230) in der zweiten Position angeordnet ist.

9. Befestigungseinheit (200) nach Anspruch 8, **dadurch gekennzeichnet, dass** der Riegel (206) einen Vorsprung (258) aufweist, dass der Vorsprung (258) mit der Vierkantscheibe (246) in Eingriff ist, so dass die Vierkantscheibe (246) den Riegel (206) in der entriegelten Position hält, wenn die Vierkantscheibe (246) in der Sperrwinkelstellung ausgerichtet ist, das Sperrelement (250) in der Sperrposition angeordnet ist und wenn der Riegel (206) mit Hilfe der Betätigungseinheit (214) über die entriegelte Position hinausbewegt worden ist, wobei die Vierkantscheibe (246) in einer Entsperrwinkelstellung ein Bewegen des Riegels (206) von der entriegelten in die verriegelte Position erlaubt, und wobei die Form des ersten Klinkenrades (242) und der Vierkantscheibe (246) derart aufeinander abgestimmt sind, dass bei einer Drehung des ersten Klinkenrades (242) um eine Klinke die Ausrichtung der Vierkantscheibe (246) jeweils zwischen der Sperrwinkelstellung und der Entsperrwinkelstellung geändert wird, wobei an dem Riegel (206) ein Kontaktelement (260) zur Kontaktierung des ersten Klinkenrades (242) vorgesehen ist, und dass das Kontaktelement (260) bei einer Betätigung des Betätigungselementes (214) jeweils das erste Klinkenrad (242) um eine Klinke entgegen der ersten Richtung (PI) dreht, und wobei das Kontaktelement (260) relativ zum Riegel (206) derart in einer Aussparung des Riegels (206) federnd gelagert ist, dass es in den Riegel (206) zumindest teilweise hineinbewegbar ist.

10. Befestigungseinheit (200) nach einem der vorhergehenden Ansprüche, **dadurch gekennzeichnet, dass** die Befestigungseinheit (200) eine Verbindungseinheit (218) zur Befestigung der Befestigungseinheit (200) an der Beinplattenaufnahme eines Operationstisches (100) umfasst, dass die Verbindungseinheit (218) einen Rastmechanismus (222) zur Befestigung umfasst, und dass die Befestigungseinheit (200) ein weiteres Betätigungselement (224), insbesondere einen Knopf, zum Entrasten des Rastmechanismus (224) aufweist.

11. Befestigungsanordnung (26, 28) zur Befestigung einer Vorrichtung (10) zum Lagern eines zu röntgenden Patienten während einer Operation an einem Operationstisch (100), mit einer operationstischseitigen Befestigungseinheit (200) nach einem der Ansprüche 1 bis 10, und mit einer vorrichtungsseitigen Befestigungseinheit (400) mit einer an der Vorrichtung (12, 14) befestigbaren Montageeinheit (406) und einem relativ zur Montageeinheit (406) um eine Drehachse drehbar an dieser befestigten Zwischenstück (404), wobei das Zwischenstück (404) ein Koppelelement (402) zum Einführen in eine komplementäre Aufnahme (204) der operationstischseitigen Befestigungseinheit (200) umfasst, wobei das Zwischenstück (404) eine Platte (414) umfasst, wobei die Platte (414) des Zwischenstücks (404) zwei im gleichen Abstand, an gegenüberliegenden Seiten zur Drehachse angeordnete Aussparungen (416, 418) umfasst, wobei die Platte (412) der Montageeinheit (406) eine Aussparung (420) umfasst, die im gleichen Abstand zur Drehachse wie die beiden Aussparungen (416, 418) des Zwischenstücks (404) angeordnet ist, und wobei die Drehachse und die Längsachse des Koppelelements (402) sich nicht schneiden.

12. Anordnung, mit einem Operationstisch (100), und einer Vorrichtung (10) zum Lagern eines zu röntgenden Patienten während einer Operation, die zwei Schienen (12, 14) umfasst, wobei an jeder Schiene (12, 14) jeweils eine vorrichtungsseitige Befestigungseinheit (400) angeordnet ist, an dem Operationstisch (100) zwei operationstischseitige Befestigungseinheiten (200) gemäß einem der Ansprüche 1 bis 10 angeordnet sind, und wobei jeweils eine Schiene (12, 14) über die an ihr angebrachte vorrichtungsseitige Befestigungseinheit (400) ausschließlich an einer der operationstischseitigen Befestigungseinheiten (200) befestigt ist, wobei die vorrichtungsseitige Befestigungseinheit mit einer an der Vorrichtung (12, 14) befestigbaren Montageeinheit (406) und einem relativ zur Montageeinheit (406) um eine Drehachse drehbar an dieser befestigten Zwischenstück (404) ausgestattet ist, wobei das Zwischenstück (404) ein Koppelelement (402) zum Einführen in eine komplementäre Aufnahme (204) der operationstischseitigen Befestigungseinheit (200) umfasst, wobei das Zwischenstück (404) eine Platte (414) umfasst, wobei die Platte (414) des Zwischenstücks (404) zwei im gleichen Abstand, an gegenüberliegenden Seiten zur Drehachse angeordnete Aussparungen (416, 418) umfasst, wobei die Platte (412) der Montageeinheit (406) eine Aussparung (420) umfasst, die im gleichen Abstand zur Drehachse wie die beiden Aussparungen (416, 418) des Zwischenstücks (404) angeordnet ist, und wobei die Drehachse und die Längsachse des Koppelelements (402) sich nicht schneiden.

13. Anordnung (1000) nach Anspruch 12, **dadurch gekennzeichnet, dass** die beiden operationstischseitigen Befestigungseinheiten (200) nicht direkt über eine Verbindungseinheit miteinander verbunden sind und/oder die beiden vorrichtungsseitigen Befestigungseinheiten (400) nicht direkt über eine Verbindungseinheit miteinander verbunden sind.

14. Anordnung nach Anspruch 11, 12 oder 13, **dadurch gekennzeichnet, dass** die Montageeinheit (406) eine Bolzenaufnahme (408) umfasst, dass das Zwischenstück einen Bolzen (410) umfasst, der in der Bolzenaufnahme (408) drehbar gelagert ist, und dass die Längsachse des Bolzens (410) die Drehachse bildet, wobei in einer ersten Montageposition sich die Aussparung (420) der Montageeinheit (406) und eine der Aussparungen (416, 418) des Zwischenstücks (404) überdecken, und dass in einer zweiten Montageposition sich die Aussparung (420) der Montageeinheit (406) mit der anderen Aussparung (416 ,418) des Zwischenstücks (404) überdeckt, und wobei die Montageeinheit (406) einen Federbolzen (422) zum Verhindern einer Drehung des Zwischenstücks (404) relativ zur Montageeinheit (406) umfasst, der an der der Platte (414) des Zwischenstücks (404) gegenüberliegenden Seite der Platte (412) der Montageeinheit (406) hinter der Aussparung (420) der Montageeinheit (406) angeordnet ist, wobei ein Bolzen des Federbolzens (422) über eine Feder des Federbolzens (422) in eine Sicherungsstellung vorgespannt ist, in der der Bolzen sich durch die sich überdeckenden Aussparungen (416, 418, 420) erstreckt, und dass der Bolzen manuell entgegen der Federkraft in eine Verdrehstellung bewegbar ist, in der das Zwischenstück (404) relativ zur Montageeinheit (406) drehbar ist.

## Claims

1. A fastening unit for an operating table for fastening a device for supporting a patient to be x-rayed during an operation on an operating table,
having a base body (202), which has a receptacle (204) for receiving a coupling element (402) of a device fastening unit (400) which is attachable to the device (12, 14),
having a locking bar (206) that is fastened to the base body (202), so that said locking bar is movable relative to the base body, wherein the locking bar (206), when in a locked position, prevents removal of the coupling element (402) from the receptacle (204), and in an unlocked position, allows removal of the coupling element (402) from the receptacle (204),
having an elastic element (208) prestressing the locking bar (206) in the locked position,
having an actuating element (214) for manual movement of the locking bar (206) out of the locked position into the unlocked position, and
having a locking mechanism (240) for holding the locking bar (206) in the unlocked position,
**characterized in that**
the locking mechanism (240) holds the locking bar (206) in the unlocked position at least as long as a coupling element (402) is arranged in the receptacle (204) and the locking bar (206) has been moved into the unlocked position with the help of the actuating element (214), until the coupling element (402) has been removed from the receptacle (204) or the actuating element (214) has been actuated again.

2. The fastening unit (200) according to claim 1, **characterized in that** the receptacle (204) is designed in a V shape, in particular having a rounded bottom area.

3. The fastening unit (200) according to claim 1 or 2, **characterized in that** the actuating element (214) comprises a tension lever (214), which is particularly rotatably fastened onto the locking bar (206), the tension lever being designed and supported in such a way that it must be pulled away from the base body (202) both for moving the locking bar (206) from the locked position into the unlocked position and also for releasing the locking mechanism (240), so that the locking bar (206) is moved back into the locked position by the elastic element (208) when the coupling element (402) is accommodated in the receptacle (204).

4. The fastening unit (200) according to any one of the preceding claims, **characterized in that** a gravity pendulum (262) is rotatably fastened onto the locking bar (206), and **in that** the gravity pendulum (262) suppresses actuation of the actuating element (214) when the fastening unit (200) is situated outside of a predetermined alignment region relative to the horizontal.

5. The fastening unit (200) according to any one of the preceding claims, **characterized in that** the end (210) of the locking bar (206) protruding into the receptacle (204) is inclined, so that the locking bar (206) is automatically moved out of the locked position in the direction (P2) of the unlocked position due to the contact with the coupling element (402) when the coupling element (402) is inserted into the receptacle (204), but it is not moved so far that the locking mechanism (240) restrains the locking bar (206).

6. The fastening unit (200) according to any one of the preceding claims, **characterized in that** the locking mechanism (240) is designed as a flip-flop mechanism.

7. The fastening unit (200) according to any one of the preceding claims, **characterized in that** the fastening unit (200) comprises a probe (230) fastened rotatably on the base body (200) and prestressed in a first position with the help of an additional elastic element (232), protruding in this position at least partially into the receptacle (204), and in a second position being moved relative to the first position, against the restoring force of the additional elastic element (232), moving out of the receptacle (204) and into the base body (202), and **in that** the probe (230) is designed so that it is automatically moved from the first position into the second position upon insertion of the coupling element (402) into the receptacle (204), and is held in the second position due to the coupling element (402) thereby accommodated until the coupling element (402) is arranged in the receptacle (204).

8. The fastening unit (200) according to claim 7, **characterized in that** the locking mechanism (240) comprises a first ratchet wheel (242), a second ratchet wheel (244) and a rectangular disk (246), which are mounted in a rotationally fixed manner on a joint shaft (248), such that the locking mechanism (240) comprises a locking element (250), which is prestressed in a locking position, such that the locking element is engaged with the second ratchet wheel (244) when in the locking position, and thereby prevents the shaft (248) from rotating in a first direction (PI), and **in that** the locking element (250) allows rotation of the shaft (248) in the first direction (PI) when in the released position, wherein the probe (230), when it is arranged in the first position, contacts the locking element (250) and holds it in the released position against its prestress, and wherein the locking element (250) is arranged in the locked position when the probe (230) is arranged in the second position.

9. The fastening unit (200) according to claim 8, **characterized in that** the locking bar (206) has a protrusion (258); **in that** the protrusion (258) is engaged with the rectangular disk (246), so that the rectangular disk (246) holds the locking bar (206) in the unlocked position when the rectangular disk (246) is aligned in the locked angle position; **in that** the locking element (250) is arranged in the locked position, and when the locking bar (206) has been moved with the help of the actuating unit (214) beyond the unlocked position, wherein when the rectangular disk (246) is in an unlocked angle position, it allows movement of the locking bar (206) from the unlocked position to the locked position, and wherein the shapes of the first ratchet wheel (242) and the rectangular disk (246) are coordinated with one another, so that with a rotation of the first ratchet wheel (242) by one ratchet, the alignment of the rectangular disk (246) is altered between the locked angle position and the unlocked angle position, wherein a contact element (260) for contacting the first ratchet wheel (242) is provided on the locking bar (206), and **in that** the contact element (260) rotates the first ratchet wheel (242) by one ratchet against the first direction (PI) when the actuating element (214) is actuated, and wherein the contact element (260) is supported elastically relative to the locking bar (206) in a recess in the locking bar (206), so that it can be moved at least partially into the locking bar (206) .

10. The fastening unit (200) according to any one of the preceding claims, **characterized in that** the fastening unit (200) comprises a connecting unit (218) for fastening the fastening unit (200) onto the leg plate receptacle of an operating table (100); **in that** the connecting unit (218) comprises a catch mechanism (222) for fastening; and **in that** the fastening unit (200) has an additional actuating element (224), in particular a button for unlocking the catch mechanism (224).

11. The fastening arrangement (26, 28) for fastening a device (10) for supporting a patient who is to be x-rayed during an operation on an operating table (100), having a fastening unit (200) on the operating table according to any one of claims 1 to 10, and having a device fastening unit (400) and having a mounting unit (406) that can be fastened on the device (12, 14) and having an intermediate piece (404) that can be fastened onto the mounting unit, so that it can be rotated about an axis of rotation relative to the mounting unit (406), wherein the intermediate piece (404) is a coupling element (402) for insertion into a complementary receptacle (204) of the fastening element (200) on the operating table, wherein the intermediate piece (404) comprises a plate (414), wherein the plate (414) of the intermediate piece (404) comprises two recesses (416, 418) arranged at the same distance on opposite sides of the axis of rotation, wherein the plate (412) of the mounting unit (406) comprises a recess (420) located at the same distance from the axis of rotation as the two recesses (416, 418) of the intermediate piece (404), and wherein the axis of rotation and the longitudinal axis of the coupling element (402) do not intersect.

12. An arrangement comprising an operating table (100) and a device (10) for supporting a patient to be x-rayed during an operation, comprising two rails (12, 14), wherein a device fastening unit (400) is arranged on each rail (12, 14), two operating table fastening units (200) being arranged on the operating table (100) according to any one of claims 1 to 10, and wherein one rail (12, 14) respectively is fastened exclusively on one of the operating table fastening units (200) by means of the device fastening unit (400) mounted on it, wherein the device fastening unit is equipped with a mounting unit (406) that can be fastened on the device (12, 14) and with an intermediate piece (404) that is attached to the mounting unit (406) in such a way that it can rotate about an axis of rotation relative to the mounting unit, wherein the intermediate piece (404) comprises a coupling element (402) for insertion into a complementary receptacle (204) of the fastening unit (200) on the operating table, wherein the intermediate piece (404) comprises a plate (414), wherein the plate (414) of the intermediate piece (404) comprises two recesses (416, 418) arranged at the same distance but on opposite sides relative to the axis of rotation, wherein the plate (412) of the mounting unit (406) comprises a recess (420) which is arranged at the same distance from the axis of rotation as the two recesses (416, 418) of the intermediate piece (404), and wherein the axis of rotation and the longitudinal axis of the coupling element (402) do not intersect.

13. The arrangement (1000) according to claim 12, **characterized in that** the two fastening units (200) on the operating table are not directly connected to one another by means of a connecting unit and/or the two fastening units (400) on the device are not connected directly to one another by means of a connecting unit.

14. The arrangement according to claim 11, 12 or 13, **characterized in that** the mounting unit (406) comprises a bolt receptacle (408); **in that** the intermediate piece comprises a bolt (410) which is rotatably mounted in the bolt receptacle (408), and **in that** the longitudinal axis of the bolt (410) forms the axis of rotation, wherein the recess (420) of the mounting unit (406) and one of the recesses (416, 418) of the intermediate piece (404) overlap in a first mounting position, and **in that** in a second mounting position, the recess (420) of the mounting unit (406) together with the other recess (416, 418) of the intermediate piece (404) overlap with one another in a second mounting position, and wherein the mounting unit (406) comprises a spring bolt (422) for preventing rotation of the intermediate piece (404) relative to the mounting unit (406), said spring bolt being disposed on the side of the plate (412) of the mounting unit (406) which is opposite the plate (414) of the intermediate piece (404) behind the recess (420) in the mounting unit (406), and wherein a bolt of the spring bolt (422) is prestressed by means of a spring of the spring bolt (422) into a secured position in which the bolt extends through the overlapping recesses (416, 418, 420), and **in that** the bolt can be moved manually into a twist-proof position in which the intermediate piece (404) can be rotated opposite the spring force relative to the mounting unit (406).

## Revendications

1. Unité de fixation pour une table d'opération permettant de fixer un dispositif destiné au positionnement d'un patient à radiographier au cours d'une opération sur une table d'opération,
ayant un corps de base (202), qui comporte un réceptacle (204) pour recevoir un élément d'accouplement (402) d'une unité de fixation de dispositif (400) qui peut être fixée au dispositif (12, 14),
ayant une barre de verrouillage (206) qui est fixée au corps de base (202), de sorte que ladite barre de verrouillage peut être déplacée par rapport au corps de base, dans lequel la barre de verrouillage (206), lorsqu'elle est dans une position verrouillée, empêche le retrait de l'élément d'accouplement (402) du réceptacle (204), et dans une position déverrouillée, permet le retrait de l'élément d'accouplement (402) du réceptacle (204),
ayant un élément élastique (208) précontraignant la barre de verrouillage (206) dans la position verrouillée,
ayant un élément d'actionnement (214) pour le déplacement manuel de la barre de verrouillage (206) hors de la position verrouillée dans la position déverrouillée, et
ayant un mécanisme de verrouillage (240) pour maintenir la barre de verrouillage (206) dans la position déverrouillée, **caractérisée en ce que**
le mécanisme de verrouillage (240) maintient la barre de verrouillage (206) dans la position déverrouillée au moins tant qu'un élément d'accouplement (402) est agencé dans le réceptacle (204) et que la barre de verrouillage (206) a été déplacée dans la position déverrouillée à l'aide de l'élément d'actionnement (214), jusqu'à ce que l'élément de d'accouplement (402) ait été retiré du réceptacle (204) ou que l'élément d'actionnement (214) ait à nouveau été actionné.

2. Unité de fixation (200) selon la revendication 1, **caractérisée en ce que** le réceptacle (204) est conçu en forme de V, en particulier ayant une zone de fond arrondie.

3. Unité de fixation (200) selon la revendication 1 ou 2, **caractérisée en ce que** l'élément d'actionnement (214) comprend un levier de tension (214) qui est fixé de manière particulièrement rotative sur la barre de verrouillage (206), le levier de tension étant conçu et supporté de telle manière qu'il doit être éloigné du corps de base (202) à la fois pour déplacer la barre de verrouillage (206) de la position verrouillée dans la position déverrouillée et également pour libérer le mécanisme de verrouillage (240), de sorte que la barre de verrouillage (206) est repoussée dans la position verrouillée par l'élément élastique (208) lorsque l'élément d'accouplement (402) est logé dans le réceptacle (204).

4. Unité de fixation (200) selon l'une quelconque des revendications précédentes, **caractérisée en ce qu'**un pendule de gravité (262) est fixé de manière rotative sur la barre de verrouillage (206), et **en ce que** le pendule de gravité (262) supprime l'actionnement de l'élément d'actionnement (214) lorsque l'unité de fixation (200) est située à l'extérieur d'une région d'alignement prédéterminée par rapport à l'horizontale.

5. Unité de fixation (200) selon l'une quelconque des revendications précédentes, **caractérisé en ce que** l'extrémité (210) de la barre de verrouillage (206) faisant saillie dans le réceptacle (204) est inclinée, de sorte que la barre de verrouillage (206) est automatiquement déplacée hors de la position verrouillée dans la direction (P2) de la position déverrouillée en raison du contact avec l'élément d'accouplement (402) lorsque l'élément d'accouplement (402) est inséré dans le réceptacle (204), mais n'est pas déplacée jusqu'à ce que le mécanisme de verrouillage (240) retienne la barre de verrouillage (206).

6. Unité de fixation (200) selon l'une quelconque des revendications précédentes, **caractérisée en ce que** le mécanisme de verrouillage (240) est conçu comme un mécanisme à bascule.

7. Unité de fixation (200) selon l'une quelconque des revendications précédentes, **caractérisée en ce que** l'unité de fixation (200) comporte une sonde (230) fixée de manière rotative sur le corps de base (200) et précontrainte dans une première position à l'aide d'un élément élastique supplémentaire (232) faisant saillie dans cette position au moins partiellement dans le réceptacle (204), et dans une seconde position étant déplacée par rapport à la première position, contre la force de rappel de l'élément élastique supplémentaire (232), quittant le réceptacle (204) et dans le corps de base (202), et **en ce que** la sonde (230) est conçue pour être automatiquement déplacée de la première position dans la seconde position lors de l'insertion de l'élément d'accouplement (402) dans le réceptacle (204), et est maintenue dans la seconde position grâce à l'élément d'accouplement (402) ainsi logé jusqu'à ce que l'élément d'accouplement (402) soit agencé dans le réceptacle (204).

8. Unité de fixation (200) selon la revendication 7, **caractérisée en ce que** le mécanisme de verrouillage (240) comprend une première roue à rochet (242), une seconde roue à rochet (244) et un disque rectangulaire (246), qui sont montés de manière fixe en rotation sur un arbre d'articulation (248), de sorte que le mécanisme de verrouillage (240) comprend un élément de verrouillage (250) qui est précontraint dans une position de verrouillage, de sorte que l'élément de verrouillage vient en prise avec la seconde roue à rochet (244) lorsqu'il est dans la position de verrouillage, et empêche ainsi la rotation de l'arbre (248) dans une première direction (PI), et **en ce que** l'élément de verrouillage (250) permet la rotation de l'arbre (248) dans la première direction (PI) lorsqu'il est dans la position libérée, dans laquelle la sonde (230), lorsqu'elle est agencée dans la première position, vient en contact avec l'élément de verrouillage (250) et le maintient dans la position libérée contre sa précontrainte, et dans laquelle l'élément de verrouillage (250) est agencé dans la position verrouillée lorsque la sonde (230) est agencée dans la seconde position.

9. Unité de fixation (200) selon la revendication 8, **caractérisée en ce que** la barre de verrouillage (206) comporte une saillie (258) ; **en ce que** la saillie (258) vient en prise avec le disque rectangulaire (246), de sorte que le disque rectangulaire (246) maintient la barre de verrouillage (206) dans la position déverrouillée lorsque le disque rectangulaire (246) est aligné dans la position angulaire verrouillée ; **en ce que** l'élément de verrouillage (250) est agencé dans la position verrouillée et lorsque la barre de verrouillage (206) a été déplacée à l'aide de l'unité d'actionnement (214) au-delà de la position déverrouillée, dans laquelle lorsque le disque rectangulaire (246) est dans une position angulaire déverrouillée, il permet le mouvement de la barre de verrouillage (206) de la position déverrouillée à la position verrouillée, et dans laquelle les formes de la première roue à rochet (242) et du disque rectangulaire (246) sont coordonnées l'une avec l'autre, de sorte qu'une rotation de la première roue à rochet (242) d'un rochet modifie l'alignement du disque rectangulaire (246) entre la position angulaire verrouillée et la position angulaire déverrouillée, dans laquelle un élément de contact (260) pour venir en contact avec la première roue à cliquet (242) est disposé sur la barre de verrouillage (206), et **en ce que** l'élément de contact (260) fait tourner la première roue à rochet (242) d'un rochet contre la première direction (PI) lorsque l'élément d'actionnement (214) est actionné, et dans laquelle l'élément de contact (260) est supporté élastiquement par rapport à la barre de verrouillage (206) dans un évidement de la barre de verrouillage (206), de sorte qu'elle peut être déplacée au moins partiellement dans la barre de verrouillage (206).

10. Unité de fixation (200) selon l'une quelconque des revendications précédentes, **caractérisée en ce que** l'unité de fixation (200) comprend une unité de raccordement (218) pour fixer l'unité de fixation (200) sur le réceptacle de plaque de pied d'une table d'opération (100) ; **en ce que** l'unité de raccordement (218) comprend un mécanisme d'accrochage (222) pour la fixation ; et **en ce que** l'unité de fixation (200) comporte un élément d'actionnement supplémentaire (224), en particulier un bouton pour déverrouiller le mécanisme d'accrochage (224).

11. Dispositif de fixation (26, 28) pour fixer un dispositif (10) destiné au positionnement d'un patient à radiographier au cours d'une opération sur une table d'opération (100), comportant une unité de fixation (200) sur la table d'opération selon l'une quelconque des revendications 1 à 10, et comportant une unité de fixation de dispositif (400) et comportant une unité de montage (406) pouvant être fixée sur le dispositif (12, 14) et comportant une pièce intermédiaire (404) pouvant être fixée sur l'unité de montage, de manière à pouvoir tourner autour d'un axe de rotation par rapport à l'unité de montage (406), dans lequel la pièce intermédiaire (404) est un élément d'accouplement (402) à insérer dans un réceptacle complémentaire (204) de l'élément de fixation (200) sur la table d'opération, dans lequel la pièce intermédiaire (404) comprend une plaque (414), dans lequel la plaque (414) de la pièce intermédiaire (404) comprend deux évidements (416, 418) agencés à la même distance sur des côtés opposés de l'axe de rotation, dans lequel la plaque (412) de l'unité de montage (406) comprend un évidement (420) situé à la même distance de l'axe de rotation que les deux évidements (416, 418) de la pièce intermédiaire (404), et dans lequel l'axe de rotation et l'axe longitudinal de l'élément d'accouplement (402) ne se croisent pas.

12. Dispositif comprenant une table d'opération (100) et un dispositif (10) destiné au positionnement d'un patient à radiographier au cours d'une opération, comprenant deux rails (12, 14), dans lequel une unité de fixation de dispositif (400) est agencée sur chaque rail (12, 14), deux unités de fixation de table d'opération (200) étant agencées sur la table d'opération (100) selon l'une quelconque des revendications 1 à 10, et dans lequel un rail (12, 14) est respectivement fixé uniquement sur l'une des unités de fixation de table d'opération (200) au moyen de l'unité de fixation de dispositif (400) montée sur celle-ci, dans lequel l'unité de fixation de dispositif est dotée d'une unité de montage (406) pouvant être fixée sur le dispositif (12, 14) et avec une pièce intermédiaire (404) qui est fixée à l'unité de montage (406) de manière à pouvoir tourner autour d'un axe de rotation par rapport à l'unité de montage, dans lequel la pièce intermédiaire (404) comprend un élément d'accouplement (402) à insérer dans un réceptacle complémentaire (204) de l'unité de fixation (200) sur la table d'opération, dans lequel la pièce intermédiaire (404) comprend une plaque (414), dans lequel la plaque (414) de la pièce intermédiaire (404) comprend deux évidements (416, 418) agencés à la même distance mais sur des côtés opposés par rapport à l'axe de rotation, dans lequel la plaque (412) de l'unité de montage (406) comprend un évidement (420) qui est agencé à la même distance de l'axe de rotation que les deux évidements (416, 418) de la pièce intermédiaire (404) et dans lequel l'axe de rotation et l'axe longitudinal de l'élément d'accouplement (402) ne se croisent pas.

13. Dispositif (1000) selon la revendication 12, **caractérisé en ce que** les deux unités de fixation (200) sur la table d'opération ne sont pas directement reliées l'une avec l'autre au moyen d'une unité de raccordement et/ou les deux unités de fixation (400) sur le dispositif ne sont pas directement reliées l'une avec l'autre au moyen d'une unité de raccordement.

14. Dispositif selon la revendication 11, 12 ou 13, **caractérisé en ce que** l'unité de montage (406) comprend un réceptacle de boulon (408) ; **en ce que** la pièce intermédiaire comprend un boulon (410) qui est monté de manière rotative dans le réceptacle de boulon (408), et **en ce que** l'axe longitudinal du boulon (410) forme l'axe de rotation, dans lequel l'évidement (420) de l'unité de montage (406) et l'un des évidements (416, 418) de la pièce intermédiaire (404) se chevauchent entre eux dans une première position de montage, et **en ce que** dans une seconde position de montage, l'évidement (420) de l'unité de montage (406) avec l'autre évidement (416, 418) de la pièce intermédiaire (404) se chevauchent entre eux dans une seconde position de montage, et dans lequel l'unité de montage (406) comprend un boulon à ressort (422) pour empêcher la rotation de la pièce intermédiaire (404) par rapport à l'unité de montage (406), ledit boulon à ressort étant disposé sur le côté de la plaque (412) de l'unité de montage (406) opposée à la plaque (414) de la pièce intermédiaire (404) derrière l'évidement (420) dans l'unité de montage (406), et dans lequel un boulon du boulon à ressort (422) est précontraint au moyen d'un ressort du boulon à ressort (422) dans une position fixe dans laquelle le boulon s'étend à travers les évidements se chevauchant (416, 418, 420), et **en ce que** le boulon peut être déplacé manuellement dans une position anti-torsion dans laquelle la pièce intermédiaire (404) peut tourner en sens contraire à la force du ressort par rapport à l'unité de montage (406).
